# EUROPEAN PATENT SPECIFICATION

(11) **EP 0 584 223 B1**
(45) Date of publication and mention of the grant of the patent: **11.08.1999**
(21) Application number: 92912076.4
(22) Date of filing: 11.05.1992
(51) Int. Cl.: C07D 207/16, C07D 211/60, C07D 401/06, C07D 409/06, C07C 235/72, A61K 31/395, A61K 31/40, A61K 31/215

(54) **NOVEL IMMUNOSUPPRESSIVE COMPOUNDS**
NEUE IMMUNSUPPRESSIVE VERBINDUNGEN
NOUVEAUX COMPOSES IMMUNOSUPPRESSEURS

(30) Priority: 09.05.1991 US 697785
(43) Date of publication of application: 02.03.1994
(73) Proprietor: VERTEX PHARMACEUTICALS INCORPORATED, Cambridge, MA 02139-4211 (US)
(72) Inventor: ARMISTEAD, David, M., Maynard, MA 01754 (US)
(74) Representative: VOSSIUS & PARTNER
(86) International application number: US9203913
(87) International publication number: WO9219593

(56) References cited:
- EP-A- 0 172 458
- EP-A- 0 384 341
- EP-A- 0 457 163
- WO-A-92/00278

## Description

### Background of the Invention

Post operative graft rejections are a major complication affecting the success of bone marrow and organ transplantations. However, through the use of immunosuppressive drug therapy, graft rejection in organ transplantation can be significantly reduced.

A wide variety of diseases can be characterized as "autoimmune diseases". Such diseases are similar to graft rejection, except that the rejection is of self tissue. Immunosuppressive therapy can also be of use in preventing this inappropriate self rejection.

One widely accepted immunosuppressant for the prevention of graft rejection is cyclosporin A (CsA). It is a natural product of fungal metabolism and has been demonstrated to have potent immunosuppressive activity in clinical organ transplantations. Calne, R.Y. et al., Br. Med. J. 282:934-936 (1981); White, D.J.C. Drugs 24:322-334 (1982). Although CsA is widely used in immunosuppressant therapy, its usage (particularly in high dosage) is often accompanied by side effects which include nephrotoxicity, hepatotoxicity and other central nervous system disorders.

The following diseases have been treated with cyclosporin A with positive results, confirming the importance of the autoimmune component in these diseases and their effective treatment with compounds working by selective T-cell immune suppression similar to cyclosporin A.
1) Ophthalmology: Uveitis, Behcet's disease and Grave's ophthalmopathy.
   Weetman, A.P. et al., Lancet 486-489 (1982). Grave's opthalmopathy.
   Nussenblatt, R.B. et al., Lancet 235-238 (1983). Uveitis.
   French-Constant, C. et al., Lancet 454 (1983). Behcet's disease.
   Sanders, M. et al., Lancet 454-455 (1983). Behcet's disease.
   Note: Cyclosporin A is currently approved in Japan for the treatment of Behcet's disease, the first autoimmune disease indication for this compound.
2) Dermatology: Various autoimmune skin diseases including psoriasis.
   Zabel, P. et al., Lancet 343 (1984). Acute dermatomyositis.
   van Joost, T. et al., Arch. Dermatol. 123:166-167 (1987). Atopic skin disease.
   Appleboom, T. et al., Amer. J. Med. 82:866-867 (1987). Scleroderma.
   Logan, R.A. and R.D.R. Camo, J. Roy. Soc. Med. 81:417-418 (1988). Eczema.
   Griffiths, C.E.M. et al., Brit. Med. J. 293:731-732 (1986). Psoriasis.
   Ellis, C.N. et al., J. Amer. Med. Assoc. 256:3110-3116 (1986). Psoriasis.
3) Hematology: Various diseases including anemia.
   Toetterman, T.H. et al., Lancet, 693 (1984). Pure red cell aplasia (PRCA).
   Stryckmans, P.A. et al., New Engl. J. Med. 310:655-656 (1984). Aplastic anemia.
   Gluckman, E. et al., Bone Marrow Transplant 3 Suppl. 1, 241 (1988). Aplastic anemia.
4) Gastroenterology/Hepatology: Primary cirrhosis, autoimmune hepatitis, ulcerative colitis, Crohn's disease and other gastrointestinal autoimmune diseases.
   Wiesner, R.H. et al., Hepatology 7:1025, Abst. #9, (1987). Primary biliary cirrhosis.
   Hyams, J.S. et al., Gastroenterology 93:890-893 (1987). Autoimmune hepatitis.
   Allison, M.C. et al., Lancet, 902-903 (1984). Crohn's disease.
   Brynskov, J. et al., Gastroenterology 92:1330 (1987). Crohn's disease.
   Porro, G.B. et al., Ital. J. Gastroenterol. 19:40-41 (1987). Ulcerative colitis.
5) Neurology: Amyotrophic lateral sclerosis (ALS, "Lou Gehrig's disease"), myasthenia gravis and multiple sclerosis.
   Appel, S.H. et al., Arch. Neurol. 45:381-386 (1988). ALS.
   Tindall, R.S.A. et al., New Engl. J. Med. 316:719-724 (1987). Myasthenia gravis. Ann. Neurol. 24, No. 1, p. 169,m Abstract P174 (1988). Multiple sclerosis.
   Dommasch, D. et al., Neurology 38 Suppl. 2, 28-29 (1988). Multiple sclerosis.
6) Nephrotic Syndrome: Nephrotic syndrome, membrano-proliferative glomerulonephritis (MPGN) and related diseases.
   Watzon, A.R. et al., Clin. Nephrol. 25:273-274 (1986). Nephrotic syndrome.
   Tejani, A. et al., Kidney Int. 33:729-734 (1988). Nephrotic syndrome.
   Meyrier, A. et al., Transplant Proc. 20, Suppl. 4 (Book III), 259-261 (1988). Nephrotic syndrome.
   LaGrue, G. et al., Nephron. 44:382-382 (1986). MPGN.
7) Rheumatoid Arthritis (RA)
   Harper, J.I. et al., Lancet 981-982 (1984). RA
   Van Rijthoven, A.W. et al., Ann. Rheum. Dis. 45:726-731 (1986). RA.
   Dougados, M. et al., Ann. Rheum. Dis. 47:127-133 (1988). RA.
8) Insulin-Dependent Diabetes Mellitus (IDDM)
   Stiller, C.R. et al., Science 223:1362-1367 (1984). IDDM.
   Assan, R. et al., Lancet, 67-71 (1985). IDDM.
   Bougneres, P.F. et al., New Engl. J. Med. 318:663-670 (1988). IDDM.
   Diabetes 37:1574-1582 (1988). IDDM.

Many veterinary diseases are also characterized as autoimmune diseases. Autoimmune diseases such as those listed above have been observed in mammals. Papa, F.O. et al., Equine Vet. J. 22:145-146 (1990) infertility of autoimmune origin in the stallion; Gorman, N.T. and L.L. Werner, Brit. Vet. J. 142:403-410, 491-497 and 498-505 (1986) immune mediated diseases of cats and dogs; George, L.W. and S.L. White, Vet. Clin. North Amer. 6:203-213 (1984) autoimmune skin diseases in large mammals; Bennett, D., In. Pract. 6:74-86 (1984) autoimmune diseases in dogs; Halliwell, R.E., J. Amer. Vet. Assoc. 181:1088-1096 (1982) autoimmune diseases in domesticated animals.

The mechanism by which CsA causes immunosuppression has been established. In vitro, CsA inhibits the release of lymphokines, such as interleukin 2 (IL-2) [Bunjes, D. et al., Eur. J. Immunol. 11:657-661 (1981)] and prevents clonal expansion of helper and cytotoxic T cells [Larsson, E. J. Immunol. 124:2828-2833 (1980)]. CsA has been shown to bind the cytosolic protein, cyclophilin, and inhibit the prolyl-peptidyl cis-trans isomerase (PPIase) activity of that protein. Fischer, G. et al., Nature 337:476-478 (1989); Takahashi, N. et al., Nature 337:473-475 (1989). The PPIases may mediate T cell activation by catalyzing the rotomerization of peptide bonds of prolyl residues.

Recently, a second natural product isolated from Streptomyces, referred to as FK-506, has been demonstrated to be a potent immunosuppresive agent. Tanaka, H. et al., J. Am. Chem. Soc. 109:5031-5033 (1987). FK-506 inhibits IL-2 production, inhibits mixed lymphocyte culture response and inhibits cytotoxic T-cell generation in vitro at 100 times lower concentration than cyclosporin A. Kino, T. et al., J. Antibiot. 15:1256-1265 (1987). FK-506 also inhibits PPIase activity, but is structurally different from CsA and binds to a binding protein (FKBP) distinct from cyclophilin. Harding, M.W. et al., Nature 341:758-760 (1989); Siekierka, J.J., Nature 341:755-757 (1989).

This invention relates to a novel class of immunosuppressive compounds having an affinity for the FK-506 binding protein (FKBP). Once bound to this protein, the immunosuppressive compounds inhibit the prolyl peptidyl cis-trans isomerase (rotamase) activity of the FKBP and lead to inhibition of T cell activation. The compounds of this invention can be used as immunosuppressive drugs to prevent or significantly reduce graft rejection in bone marrow and organ transplantations and in the treatment of autoimmune disease in humans and other mammals.

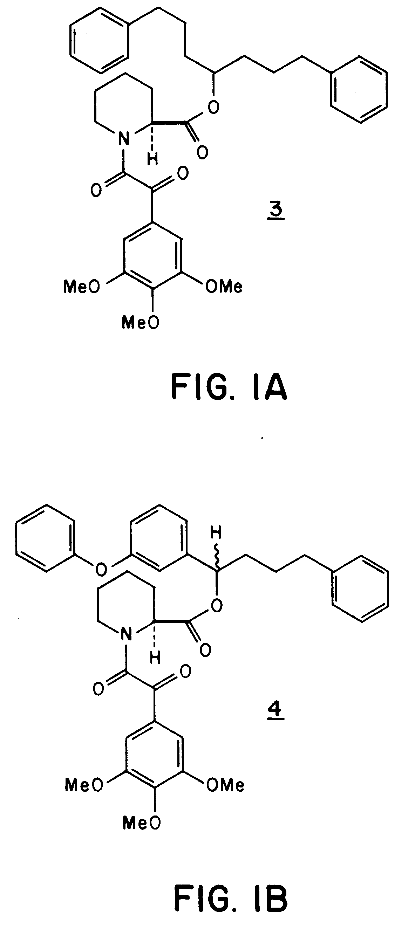

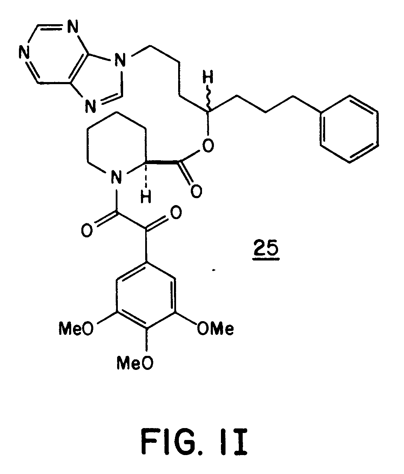

Figures 1A-1I illustrate some preferred compounds of this invention. The synthesis of each of the preferred compounds is described in detail in the Example section.

This invention relates to a novel class of immunosuppressive compounds represented by the formula I: 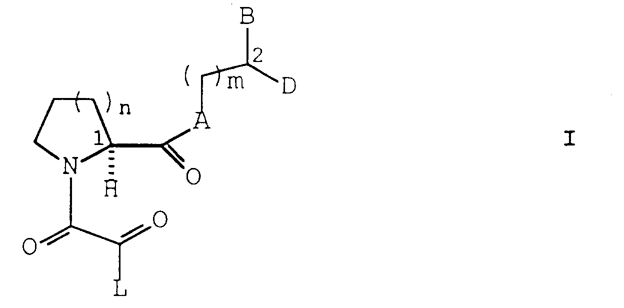 and pharmaceutically acceptable salts thereof,
wherein A is CH₂, oxygen, NH, or N-(C1-C4 alkyl);
wherein B and D are independently Ar, (C5-C7)-cycloalkyl substituted (C1-C6)-straight or branched alkyl or (C2-C6)-straight or branched alkenyl, (C5-C7)-cycloalkenyl substituted (C1-C6)-straight or branched alkyl or (C2-C6)-straight or branched alkenyl, or Ar substituted (C1-C6)-straight or branched alkyl, or Ar-substituted (C2-C6)-straight or branched alkenyl, wherein in each case, one or two carbon atoms of the straight or branched alkyl or alkenyl chains may be substituted with 1-2 heteroatoms selected from the group consisting of oxygen, sulfur, SO and SO₂ in chemically reasonable substitution patterns, or 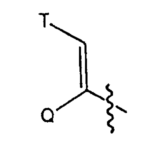
wherein Q is hydrogen, (C1-C6)-straight or branched alkyl or (C2-C6)-straight or branched alkenyl;
wherein T is Ar or substituted 5-7 membered cycloalkyl with substituents at positions 3 and 4 which are independently selected from the group consisting of oxo, hydrogen, hydroxyl, O-(C1-C4)-alkyl and O-(C2-C4)-alkenyl;
wherein Ar is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, monocyclic and bicyclic heterocyclic ring systems with individual ring sizes being 5 or 6 which may contain in either or both rings a total of 1-4 heteroatoms independently selected from oxygen, nitrogen and sulfur; wherein Ar may contain one to three substituents which are independently selected from the group consisting of hydrogen, halogen, hydroxymethyl, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl and phenyl;
wherein L is U;
wherein U is O-(C1-C4)-straight or branched alkyl, O-(C2-C4)-straight or branched alkenyl, (C1-C6)-straight or branched alkyl, (C2-C6)-straight or branched alkenyl, (C5-C7)-cycloalkyl, (C5-C7)-cycloalkenyl substituted with (C1-C4)-straight or branched alkyl, (C5-C7)-cycloalkenyl substituted (C2-C4)-straight or branched alkenyl, [(C1-C4)-alkyl or (C2-C4)-alkenyl]-Ar or Ar (Ar as described above);
wherein n is 1 or 2; and
wherein m is 0 or 1.

The stereochemistry at position 1 (Formula I) is (R) or (S), with (S) preferred. The stereochemistry at position 2 is (R) or (S).

The compounds of the present invention can be used in the form of salts derived from inorganic or organic acids and bases. Included among such acid salts are the following: acetate, adipate, alginate, aspartate, benzoate, benzenesulfonate, bisulfate butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate and undecanoate. Base salts include ammonium salts, alkali metal salts such as sodium and potassium salts, alkaline earth metal salts such as calcium and magnesium salts, salt with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

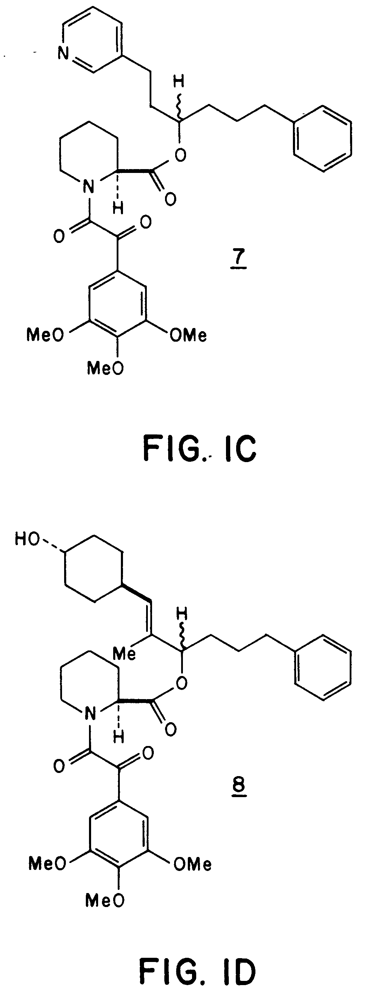

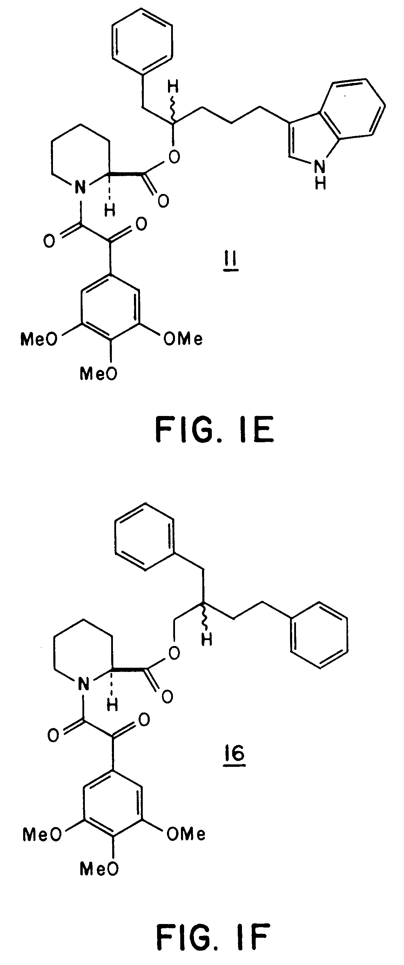

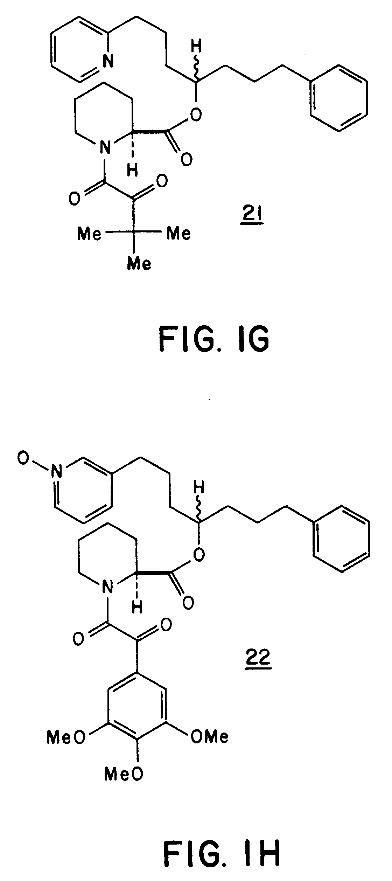

Preferably, the compounds will have a molecular weight below about 750 atomic mass units (a.m.u.) and most preferably below about 500 a.m.u. Examples of compounds in which the J and K substituents are taken together to form a heterocyclic ring are shown in Table 1 and Figure 1.

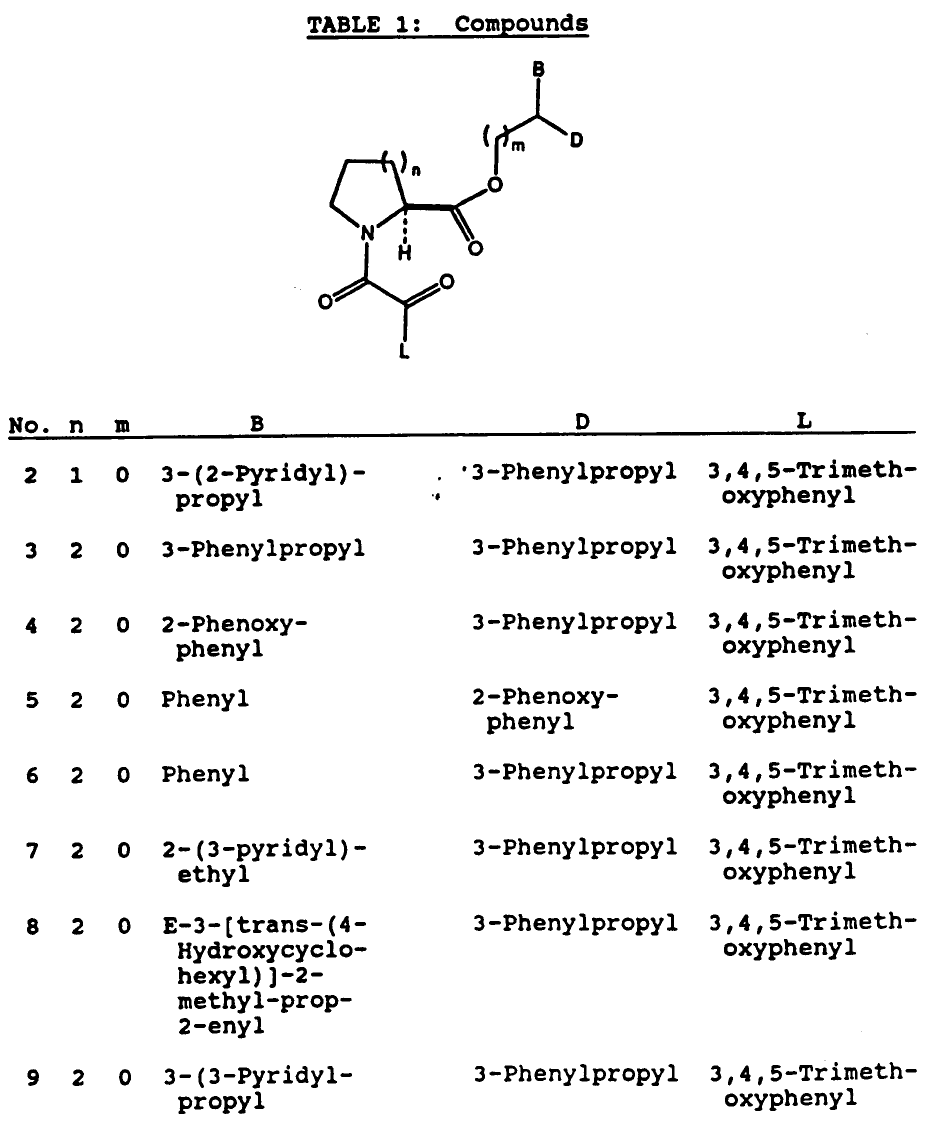

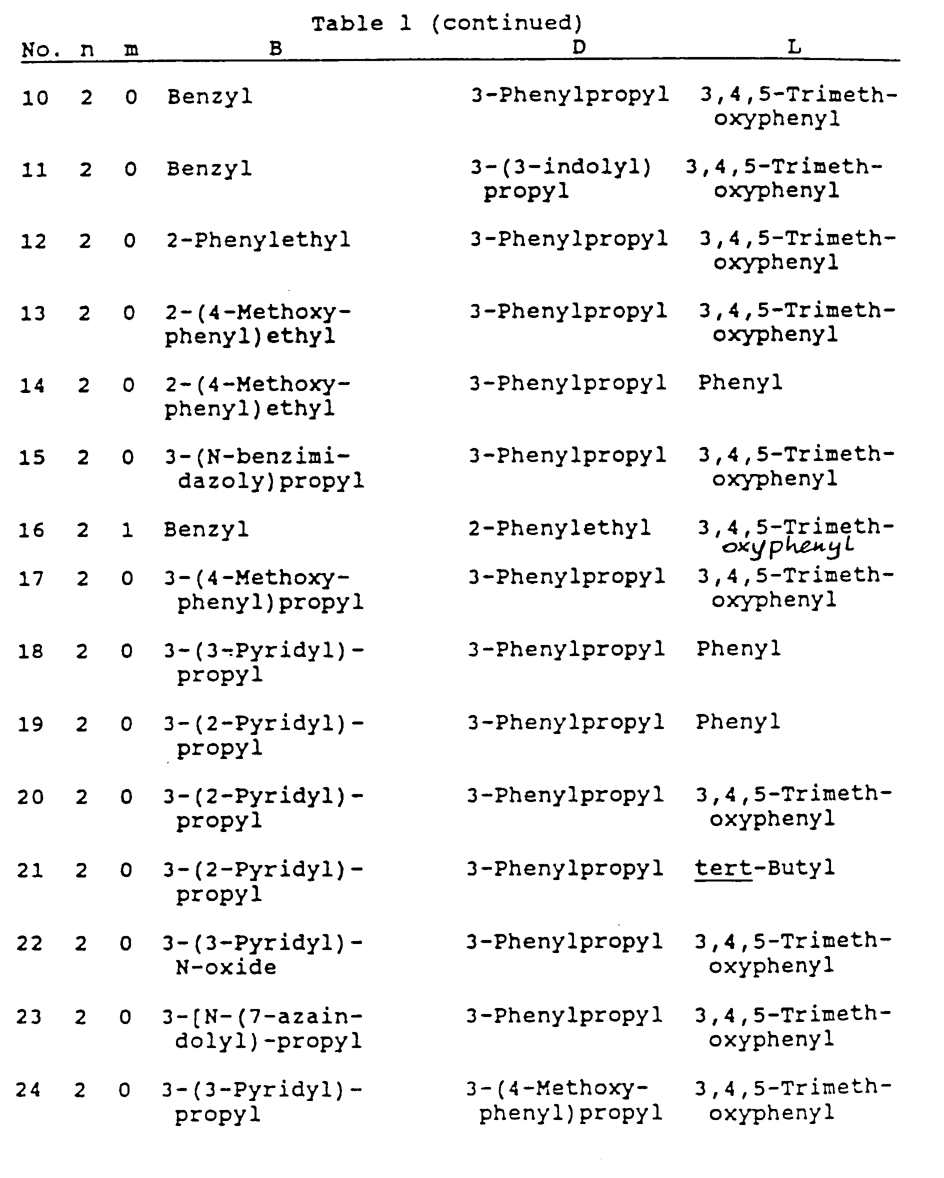

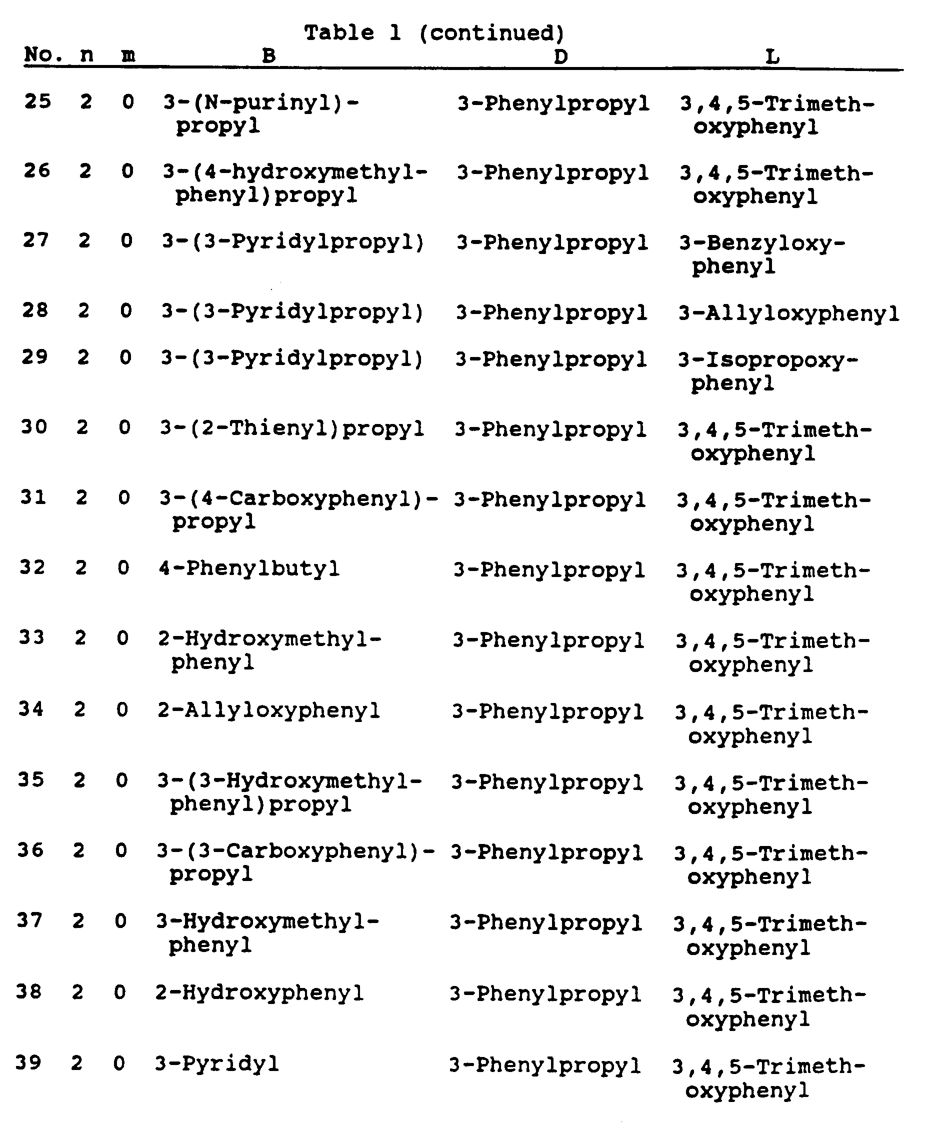

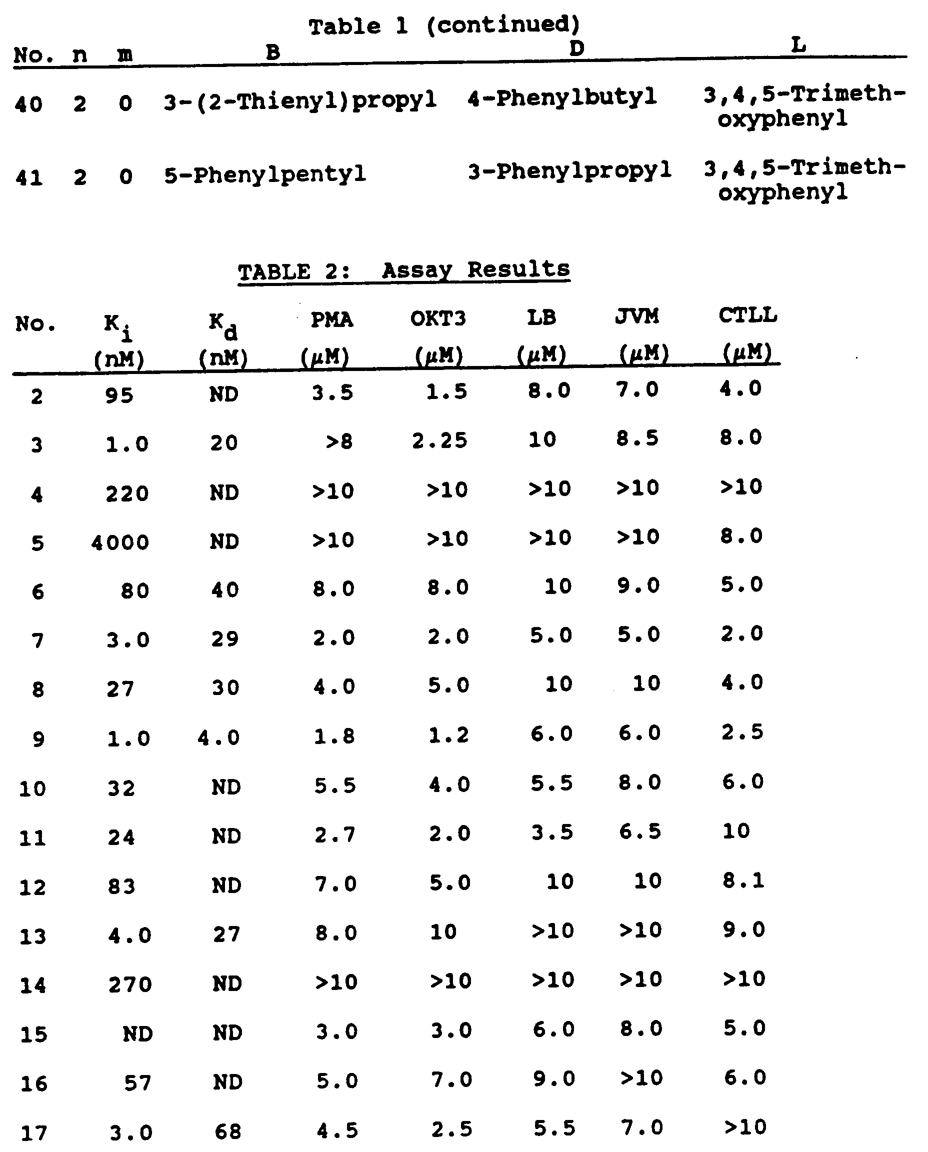

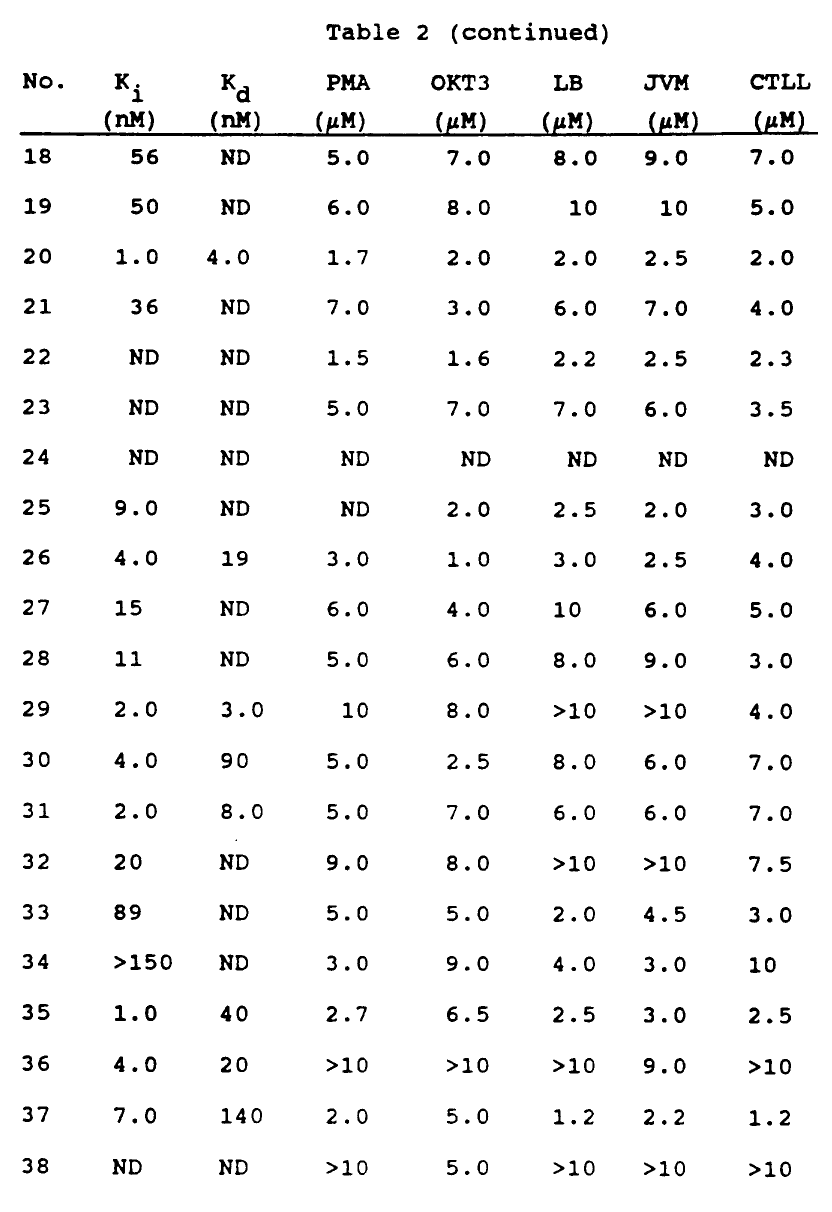

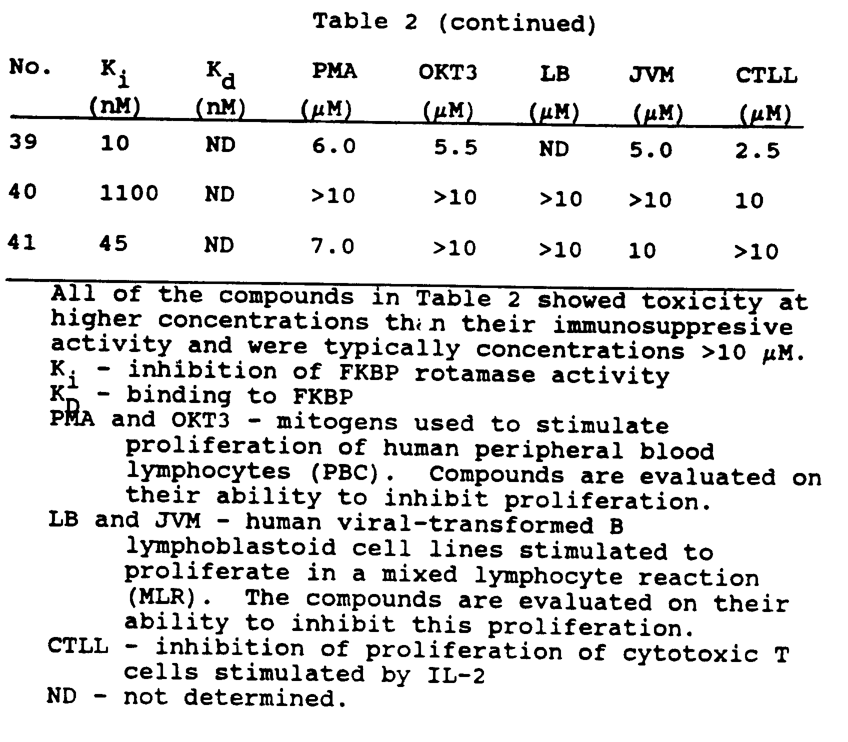

The immunosuppressive compounds of this invention have an affinity for the FK-506 binding protein which is located in the cytosol of lymphocytes, particularly T lymphocytes. When the immunosuppressive compounds are bound to the FKBP, they act to inhibit the prolyl-peptidyl cis-trans isomerase activity of the binding protein and inhibit lymphocyte activation mediated by FKBP. One particular FK-506 binding protein has been identified by Harding, M.W. et al., Nature 341:758-760 (1989) and can be used as the standard by which to evaluate binding affinity of the compounds for FKBP. Compounds of this invention, however, may have an affinity for other FK-506 binding proteins. Inhibition of the prolyl peptidyl cis-trans isomerase may further be indicative of binding to an FK-506 binding protein.

Human FK-506 binding protein can be obtained as described by Harding, M.W. et al., Nature 341:758-760 (1989). Values for the apparent K_{d} can be determined from a competitive LH-20 binding assay performed as described by Harding et al., using 32-[1-¹⁴C]-benzoyl FK-506 as a reporting ligand; or using [³H]dihydro-FK-506, as described by Siekierka, J.J. et al., Nature 341:755-757 (1989). The binding affinities for several compounds of this invention for the FKBP are reported in Table 2. The data was obtained using the latter method, where the ability of an unlabeled compound to compete with the binding of [³H]dihydro-FK-506 to FK-506 binding protein was measured.

The inhibition of the PPIase (rotamase) enzyme activity of the FKBP (apparent "Kᵢ" values) can also be measured according to the methods described by either Harding, M.W. et al., Nature 341:758-760 (1989) or Siekierka, J.J. et al., Nature 341:755-757 (1989). The cis-trans isomerization of the proline-alanine peptide bond in a model substrate, N-succinyl-Ala-Ala-Pro-Phe-p-nitroanilide, is monitored spectrophotometrically in a coupled assay with chymotrypsin, which releases 4-nitroanilide from the trans form of the substrate. Fischer, G. et al., Nature 337:476-478 (1989). The inhibitory effect of the addition of different concentrations of inhibitor on the extent of the reaction is determined, and analysis of the change in first order rate constant as a function of inhibitor concentration yields an estimate of the apparent Kᵢ value. The extent of enzyme inhibition (Kᵢ) of some preferred compounds is shown in Table 2.

The compounds of the present invention can be further characterized in cellular biological experiments in vitro where their resemblance in function and use to cyclosporin A and to FK-506 is apparent. (See Table 3).

**TABLE 3**

| Assays and IC₅₀ Value for Drugs | Cyclosporin A | Rapamycin | FK-506 |
|---|---|---|---|
| 1) Human PBL + OKT3 | <1µg/ml | <1µg/ml | <1µg/ml |
| 2) T-Cell Hybridoma + TCR/CD2 | <1µg/ml | <1µg/ml | <1µg/ml |
| 3) Apoptosis | Blocks at 1µg/ml | Inactive at 1µg/ml | Blocks at 1µg/ml |
| 4) CTLL Proliferation + IL-2 | >>1µg/ml | ≃0.01µg/ml | >>1µg/ml |

| | | | |
|---|---|---|---|
| 1) Assay similar to Yoshimura, N. et al., Transplantation 47:356-359 (1989). Assay uses fresh human peripheral blood lymphocytes isolated by Ficoll-Hypaque density centrifugation, stimulated by the OKT3 antibody (anti-CD3) which stimulates via interaction with CD3. Stimulation is measured by incorporation of radioactive thymidine [(³H)TdR] into proliferating cells, with an uninhibited control signal of 48,000-75,000 cpm. IC₅₀ values are estimated from inhibition of proliferation observed at various drug concentrations. | | | |
| 2) Assay similar to above, but using T-cell clone stimulated with antibody to the T-cell receptor (TCR) and antibody to CD2. Stimulation is measured by incorporation of radioactive thymidine [(³H)TdR] into proliferating cells, with an uninhibited control signal of 23,000 cpm. IC₅₀ values are estimated from inhibitions of proliferation observed at various drug concentrations. | | | |
| 3) Assay according to Shi, Y. et al., Nature 339:625-626 (1989). The assay uses a T-cell hybridoma similar to that described. The assay measures activation-induced (anti-CD3) cell death (evaluated by counting viable cells after staining as described) in a T-cell hybridoma that mimics the effect known to occur in immature thymocytes. The ability of cyclosporin A and FK-506 to inhibit this cell death is herein used as a sensitive indication of compounds with cyclosporin-like and/or FK-506-like mechanism of action. Note that the chemically related, but mechanistically distinct, immunosuppressant rapamycin is inactive in this assay. | | | |
| 4) Assay according to DuMont, F. et al., J. Immunol. 144:251-258 (1990). The assay measures the stimulation of CTLL cells in response to IL-2. Proliferation is measured by incorporation of (³H)TdR. Immunosuppressants which work by a similar mechanism to cyclosporin A and FK-506 will not inhibit in this IL-2 driven process, since they function by the inhibition of production of endogenous IL-2. In this assay, exogenous IL-2 is provided to overcome this block. Note that the chemically related, but mechanistically distinct immunosuppressant, rapamycin, is active in this assay. | | | |

These assays and the ones set forth in the Example Section can be used to profile the cellular activity of the compounds of the present invention. Thus, it is clear from these results that the compounds of this invention resemble both cyclosporin A and FK-506 in its cellular activity, including immunosuppression, in contrast to the mechanistically dissimilar immunosuppressant agent rapamycin. Furthermore, the observed cellular activity is consistent quantitatively with the activity observed for FKBP binding and inhibition of PPIase (rotamase) activity shown in Table 2. Thus, the compounds can be used as immunosuppressants for prophylaxis of organ rejection or treatment of chronic graft rejection and for the treatment of autoimmune diseases.

The immunosuppressive compounds of this invention can be periodically administered to a patient undergoing bone marrow or organ transplantation or for another reason in which it is desirable to substantially reduce or suppress a patient's immune response, such as in various autoimmune diseases. The compounds of this invention can also be administered to mammals other than humans for treatment of various mammalian autoimmune diseases.

The novel compounds of the present invention possess an excellent degree of activity in suppression of antigen-stimulated growth and clonal expansion of T-cells, especially those T-cells characterized as "helper" T-cells. This activity is useful in the primary prevention of organ transplant rejection, in the rescue of transplanted organs during a rejection episode, and in the treatment of any of several autoimmune diseases known to be associated with inappropriate autoimmune responses. These autoimmune diseases include: uveitis, Behcet's disease, Graves ophthalmopathy, psoriasis, acute dermatomyositis, atopic skin disease, scleroderma, eczema, pure red cell aplasia, aplastic anemia, primary cirrhosis, autoimmune hepatitis, ulcerative colitis, Crohn's disease, amyotrophic lateral sclerosis, myasthenia gravis, multiple sclerosis, nephrotic syndrome, membrano-proliferative glomerulonephritis, rheumatoid arthritis and insulin-dependent diabetes mellitus. In all of the above-listed autoimmune diseases, treatment is effective to reduce the symptoms and slow progression of the disease. In the case of insulin-dependent diabetes mellitus, treatment as described below is most effective when instituted before the complete cessation of natural insulin production and transition to complete dependence on external insulin.

For these purposes the compounds of the present invention are manufactured as a medicament which may be administered orally, parenterally, by inhalation spray, topically, rectally, nasally, buccally, vaginally or via an implanted reservoir in dosage formulations containing conventional non-toxic pharmaceutically-acceptable carriers, adjuvants and vehicles. The term parenteral as used herein includes subcutaneous, intravenous, intramuscular, intrasternal and intracranial injection or infusion techniques.

The pharmaceutical compositions may be in the form of a sterile injectable preparation, for example as a sterile injectible aqueous or oleagenous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or di-glycerides. Fatty acids such as oleic acid and its glyceride derivatives find use in the preparation of injectables, as do natural pharmaceutically-acceptable oils, such as olive oil or castor oil, especially in their polyoxyethylated versions. These oil solutions or suspensions may also contain a long-chain alcohol diluent or dispersant such as Ph. Helv or similar alcohol.

The compounds are manufactured as a medicament which may be administered orally, in the form of capsules or tablets, for example, or as an aqueous suspension or solution. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening and/or flavoring and/or coloring agents may be added.

The compounds of this invention are manufactured as a medicament which may also be administered in the form of suppositories for rectal administration of the drug. These compositions can be prepared by mixing the drug with a suitable non-irritating excipient which is solid at room temperature but liquid at the rectal temperature and therefore will melt in the rectum to release the drug. Such materials include cocoa butter, beeswax and polyethylene glycols.

The compounds of this invention are manufactured as a medicament which may also be administered topically, especially when the conditions addressed for treatment involve areas or organs readily accessible by topical application, including autoimmune diseases of the eye, the skin, or the lower intestinal tract. Suitable topical formulations are readily prepared for each of these areas.

For ophthalmic use, the compounds can be formulated as micronized suspensions in isotonic, pH adjusted sterile saline, or, preferably, as solutions in isotonic, pH adjusted sterile saline, either with or without a preservative such as benzylalkonium chloride. Alternatively for the ophthalmic uses, the compounds may be formulated in an ointment such as petrolatum.

For application topically to the skin, the compounds can be formulated in a suitable ointment containing the compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, the compounds can be formulated in a suitable lotion or cream containing the active compound suspended or dissolved in, for example, a mixture of one or more of the following: mineral oil, sorbitan monostearate, polysorbate 60, cetyl esters wax, cetearyl alcohol, 2-octyldodecanol, benzyl alcohol and water.

Topical application for the lower intestinal tract can be effected in a rectal suppository formulation (see above) or in a suitable enema formulation.

Dosage levels on the order of 0.01 to 100 mg/kg per day of the active ingredient compound are useful in the treatment of the above conditions. The amount of active ingredient that may be combined with the carrier materials to produce a single dosage form will vary depending upon the host treated and the particular mode of administration.

It is understood, however, that a specific dose level for any particular patient will depend upon a variety of factors, including the activity of the specific compound employed, the age, body weight, general health, sex, diet, time of administration, rate of excretion, drug combination and the severity of the particular disease being treated.

The compound can be manufactured as a medicament which is administered in combination with a steroid, such as methyl prednisolone acetate, for additional immunosuppressive effect. The steroid is manufactured as a medicament which is administered orally, intravenously, rectally, topically or by inhalation. Dosages (based upon methyl prednisolone acetate) of 0.1-5 mg/kg/day may be employed. An initial loading dose of 100-500 mg may be employed. Steroid doses may be decreased with time from the higher toward the lower doses as the clinical situation indicates.

The compounds are manufactured as a medicament which can be administered with other immunosuppressant drugs, such as rapamycin, azathioprine, 15-deoxyspergualin, cyclosporin, FK-506 or combinations of these, to increase the immunosuppressive effect. Administration of cyclosporin and FK-506 together should be avoided due to contraindications reported resulting from coadministration of these immunosuppressants. The dosage level of other immunosuppressant drugs will depend upon the factors previously stated and the immunosuppressive effectiveness of the drug combination.

OKT3, which is a murine monoclonal antibody to CD3 surface antigen of human T lymphocytes, can also be manufactured as a medicament with compounds of the present invention which is administered intravenously for rescue and reversal of acute allograft rejections, particularly in renal transplantations.

The invention will be further illustrated by way of the following examples, which are not intended to be limiting in any way.

### EXAMPLES

### General

Proton nuclear magnetic resonance (¹H NMR) spectra were recorded at 500 MHz on a Bruker AMX 500. Chemical shifts for proton resonances are reported in parts per million (δ) relative to Me₄Si (δ 0.0). Analytical high performance liquid chromatography (HPLC) was performed on either a Waters 600E or a Hewlett Packard 1050 liquid chromatograph.

The compounds described below are illustrated in Figure 1.

### EXAMPLE 1

### Synthesis of (S)-1,7-Diphenyl-4-heptanyl N-(3,4,5-trimethoxyphenylglyoxyl)pipecolate (3)

### 4-Phenyl-1-butyraldehyde (26)

To a solution of 3.2 mL (20.8 mmol) of 4-phenyl-1-butanol (Aldrich Chemical Co.) in 20 mL of CH₂Cl₂ at 0°C was added 3.2 g of powdered 3 Å molecular sieves and then 5.37 g (24.9 mmol) of pyridinium chlorochromate (PCC). The resulting suspension was stirred at 0°C for 1 h at which time an additional 2.16 g (10.0 mmol) of PCC was added and the reaction mixture was warmed to room temperature. After stirring at ambient temperature for 0.5 h, the reaction mixture was diluted with ether and filtered through celite to give 2.5 g of the crude product. Flash chromatography (elution with 5% ethyl acetate in hexane) yielded 700 mg of the aldehyde (26). ¹H NMR consistent with the product.

### 3-Phenyl-1-propylmagnesium bromide (27)

To a suspension of 736 mg (30.3 mmol) of magnesium turnings in 50 mL of THF at room temperature was added 50 µL of 1,2-dibromoethane followed by the dropwise addition of 5.5 g (25.1 mmol) of 1-bromo-3-phenylpropane (Aldrich Chemical Co.). After stirring at room temperature for 0.5 h, the supernatent was transfered via canula to a 100 mL storage vessel and subsequently used as a 0.5 M THF solution of the Grignard reagent (27).

### 1,7-Diphenyl-4-heptanol (28)

To a solution of 700 mg (4.7 mmol) of 4-phenyl-1-butanal (26) in 5.0 mL of THF at 0°C was added 10.0 mL (5.0 mmol) of 3-phenyl-1-propylmagnesium bromide (27) and the resulting mixture was stirred at 0°C for 0.5 h. The mixture was then quenched by the dropwise addition of saturated NH₄Cl and diluted with ether. The phases were separated and the organic layer was washed with water and brine and then dried over MgSO₄. Concentration gave 1.12 g of the alcohol (28) as an oil. The ¹H NMR spectrum of this compound was consistent with the structure.

### (S)-Boc-Pipecolyl-1,7-dipenyl-4-heptanyl ester (29)

To a solution of 164.2 mg (0.72 mmol) (S)-Boc-pipecolic acid in 5.0 mL of CH₂Cl₂ at room temperature was added 174.7 mg (0.65 mmol) of alcohol (28), 140.8 mg (0.72 mmol) of 1-(3-dimethylaminopropyl)3-ethylcarbodiimide hydrochloride (EDC) and a catalytic amount of N,N-dimethylaminopyridine (DMAP). The reaction mixture was stirred at ambient temperature for 0.5 h and then applied directly to a silica gel column. Elution with 10% ethyl acetate in hexane afforded 76.2 mg of the ester (29) as an oil. ¹H NMR consistent with the product.

### (S)-1,7-Diphenyl-4-heptanylpipecolate (30)

To a solution of 47 mg (0.10 mmol) of (29) in 1.0 mL of CH₂Cl₂ at ambient temperature was added 1.0 mL of trifluoroacetic acid. After stirring at room temperature for 0.5 h, the resulting solution was neutralized by the dropwise addition of saturated K₂CO₃. The layers were separated and the organic phase was washed with water, dried over MgSO₄ and concentrated to yield 23 mg of the amine (30) as an oil. ¹H NMR consistent with structure.

### 3,4,5-Trimethoxybenzoylformic acid (31)

To a solution of 9.2 g (43.4 mmol) of 3,4,5-trimethoxyacetophenone (Aldrich Chemical Co.) in 35 mL of pyridine was added 6.3 g (56.7 mmol) of selenium dioxide and the resulting solution was heated at reflux overnight. The reaction mixture was cooled to room temperature, filtered through celite and concentrated to yield a dark brown oil which was dissolved into ethyl acetate and washed with 1.0 N HCL and then with saturated NaHCO₃. The basic aqueous layer was diluted with ether and acidified with concentrated HCl. The layers were separated and the organic phase was washed with brine and then dried over Na₂SO₄ to give 8.4 g of a dark yellow solid. Recrystalization of this material from ethyl acetate-hexane then gave 6.8 g of the acid (31) as a pale yellow solid. ¹H NMR was consistent with the structure.

### (S)-1,7-Diphenyl-4-heptanyl N-(3,4,5-trimethoxyphenylglyoxyl)pipecolate (3)

To a solution of 23 mg (0.06 mmol) of the amine (30) in 1.0 mL of CH₂Cl₂ at room temperature was added 21.8 mg (0.09 mmol) of the acid (31) and then 17.9 mg (.09 mmol) of EDC and the resulting solution was stirred at room temperature for 0.5 h and applied directly to a silica gel column. Elution with 15% ethyl acetate in hexane gave 8.4 mg of the amide (3) as a mixture of rotamers. ¹H NMR (500 MHz, CDCl₃)δ 7.35-7.06(m), 5.32(br s),5.00(br s), 4.88(br s), 4.58(d), 4.31(br s), 3.95(s), 3.90(s), 3.89(s), 3.85(s), 3.44(d), 3.21(t), 3.04(t), 2.54(br s), 2.51 (br s), 2.42(br s), 2.30(d), 2.15(d), 1.83-1.21(m).

### EXAMPLE 2

### Synthesis of (R and S)-1-(3-Phenoxy)phenyl-4-phenyl-1-butyl (S)-N-(3,4,5-trimethoxyphenylglyoxyl)pipecolate (4)

### 3-Phenoxybenzaldehyde (32)

To a solution of 1.8 mL (10.3 mmol) of 3-phenoxybenzyl alcohol (Aldrich Chemical Co.) in 20 mL of CH₂Cl₂ at room temperature was added 1.5 g of powdered 4 Å molecular sieves and 2.5 g of activated MnO₂. The resulting suspension was stirred at room temperature for 0.5 h at which time an additional 2.5 g of MnO₂ was added. After stirring at room temperature for 0.5 h the reaction mixture was filtered through celite to give 1.84 g of the aldehyde (32) as an oil. ¹H NMR consistent with structure.

### (R and S)-1-(3-Phenoxy)phenyl-4-phenyl-1-butanol (33)

The alcohol (33) was prepared from 190 mg (0.96 mmol) of aldehyde (32) and 2.0 mL (1.0 mmol) of (27) in 2.0 mL of THF as described above for the synthesis of (28) in Example 1. Flash chromatography (elution with 10% ethyl acetate in hexane) afforded 108 mg of the racemic alcohol (33). ¹H NMR consistent with structure.

### (S)-N-3,4,5-(Trimethoxyphenyl)glyoxylpipecolic acid (34)

To a slurry of 953.3 mg (3.4 mmol) of the tartrate salt of (S)-pipecolic acid (Egbertson, M. and S.J. Danishefsky, J. Org. Chem. 54:11 (1989)) in 7.0 mL of CH₂Cl₂ at 0°C was added 3.9 mL (22.4 mmol) of diisopropylethylamine and 2.4 mL (18.9 mmol) of chlorotrimethylsilane and the resulting solution was allowed to stir at 0°C for 0.5 h. In a separate reaction flask 450 µL (5.2 mmol) of oxalyl chloride and three drops of DMF was added to a solution of 820 mg (3.4 mmol) of acid (31) in 7.0 mL of CH₂Cl₂. After the evolution of gas ceased, the entire contents of the second flask were added to the first reaction vessel and the resulting mixture was allowed to stir at room temperature for 1 h. The reaction mixture was concentrated, dissolved into ether and washed with 0.5 N HCl and then saturated NaHCO₃. The basic aqueous phase was acidified with concentrated HCl and extracted with ether. The ethereal extracts were washed with water, brine, dried over MgSO₄ and concentrated to give 490 mg of the acid (34). ¹H NMR consistent with structure.

### (R and S)-1-(3-Phenoxy)phenyl-4-phenyl-1-butyl (S)-N-(3,4,5-trimethoxyphenylglyoxyl)pipecolate (4)

To a solution of 29.4 mg (0.08 mmol) of acid (34) in 2.0 mL of CH₂Cl₂ at room temperature was added 11 µL (0.13 mmol) of oxalyl chloride and three drops of DMF and the reaction mixture was allowed to stir at room temperature for 0.5 h and was then concentrated and suspended in 1.0 mL of benzene. To this suspension was added 32.0 mg (0.1 mmol) of alcohol (33) and 13.4 mg (0.1 mmol) of silver cyanide. The resulting mixture was heated at reflux overnight, cooled to room temperature and concentrated. Flash chromatography (elution with 10% ethyl acetate in hexane) gave 8.8 mg of the ester (4) as a mixture of diastereomers. ¹H NMR (500 MHz, CDCl₃)δ 7.34-7.19(m), 7.18-7.03(m), 7.02-6.84(m), 6.83-6.72(m), 5.73(q), 5.69-5.55(m), 5.38(t), 4.55(br d), 4.35(dd), 3.94(s), 3.92(s), 3.89(s), 3.83(s), 3.73(s), 3.63(s), 3.48-3.35(m), 3.20(t), 3.10(t), 2.60(q), 2.40(dd), 1.95-1.91(m), 1.90-1.45(m).

### EXAMPLE 3

### Synthesis of (R and S)-6-Phenyl-1-(3-pyridyl)-3hexyl (S)-N-(3,4,5-trimethoxyphenylglyoxyl)pipecolate (7)

### 3-(3-Pyridyl)-1-propylaldehyde (35)

To a solution of 2.3 g (5.46 mmol) of the Dess-Martin periodinane (Dess, D.B. and J.C. Martin, J. Org. Chem. 48:4155 (1983)) in 10 mL of CH₂Cl₂ at 0°C was added 470 µL (3.65 mmol) of 3-(3-pyridyl)-1-propanol and the resulting mixture was allowed to warm from 0°C to ambient temperature over a 1.5 h period. To this solution was added 6.0 g (38.22 mmol) of Na₂S₂O₃ in saturated NaHCO₃ and the reaction mixture was allowed to stir at room temperature for 15 min. The reaction was extracted with CH₂Cl₂, dried over MgSO₄ and concentrated. Flash chromatography (elution with 3:1 hexane:acetone) yielded the product aldehyde (35) as an oil. ¹H NMR consistent with structure.

### (R and S)-6-Phenyl-1-(3-pyridyl)-3-hexanol (36)

The alcohol (36) was prepared from 125 mg (0.92 mmol) of aldehyde (35) and 2.0 mL (1.0 mmol) of (27) in 2.0 mL of THF as described above for the synthesis of (28) in Example 1 to give 221 mg of the crude alcohol (36). ¹H NMR consistent with structure.

### (S)-Boc-Pipecolyl-(R and S)-6-Phenyl-1-(3-pyridyl)-3-hexyl ester (37)

The ester (37) was prepared from 125 mg (0.49 mmol) of alcohol (36), 93 mg (0.41 mmol) of (S)-Boc-pipecolic acid, 94 mg (0.49 mmol) of EDC and a catalytic amont of DMAP in 1.0 mL of CH₂Cl₂ and 1.0 mL of DMF as described above for the synthesis of (29) in Example 1. Flash chromatography (elution with 2:1 hexane:ethyl acetate) gave 105 mg of the diastereomeric ester (37) as an oil. ¹H NMR consistent with structure.

### (R and S)-6-Phenyl-1-(3-pyridyl)-3-hexyl (S)-pipecolate (38)

The amine (38) was synthesized by treating 95 mg (0.20 mmol) of the ester (37) with 1.0 mL of trifluoroacetic acid in 3.0 mL of CH₂Cl₂ as described above for the preparation of (30) in Example 1 giving 58 mg of the diastereomeric amine (38) as an oil. ¹H NMR consistent with structure.

### (R and S)-6-Phenyl-1-(3-pyridyl)-3-hexyl (S)-N-(3,4,5-trimethoxyphenylglyoxyl)pipecolate (7)

The ester (7) was prepared from 54 mg (0.15 mmol) of the amine (38), 50 mg (0.22 mmol) of the acid (31) and 42 mg (0.22 mmol) of EDC in 3.0 mL of CH₂Cl₂ as described above in the synthesis of ester (3) in Example 1. Flash chromatography (elution with 1:1 ethyl acetate:hexane) gave 73 mg of the diasteromeric ester (7) as a mixture of rotamers. ¹H NMR (500 MHz CDCl₃)δ 8.48-8.42(m), 7.50-7.41(m), 7.32(d), 7.27-7.03(m), 5.38(d), 5.31(d), 5.06-5.01(m), 4.97-4.93(m), 4.60(br d), 3.92(s), 3.88(s), 3.86(s), 3.84(s), 3.82(s), 3.79(s), 3.46(br d), 3.27(br t), 2.73-2.68(m), 2.38-2.29(m), 1.98-1.76(m), 1.75-1.60(m), 1.56-1.51(m), 1.38-1.20(m).

### EXAMPLE 4

### Synthesis of (R and S)-(E)-1-[trans-(4-Hydroxycyclohexyl)]-2-methyl-6-phenyl-3-hex-1-enyl (S)-N-(3,4,5-trimethoxyphenylglyoxyl)pipecolate (8)

### cis- and trans-4-(tert-Butyldimethylsilyloxy) cyclohexan-1-ol (39) and (40)

To a solution of 3.43 g (21.7 mmol) of cis- and trans-methyl 4-hydroxycyclohexane carboxylate (Noyce, D.S. and D.B. Denney, J. Am. Chem. Soc. 74:5912 (1952)) in 45 mL of methylene chloride at 0°C was added 3.0 mL (26.0 mmol) of 2,6-lutidine followed by 5.5 mL (23.8) mmol of tert-butyldimethylsilyl trifluoromethanesulfonate. The ice bath was removed and the reaction mixture was allowed to stir at 25°C for 2 h at which time the solution was poured into saturated sodium bicarbonate. The layers were partitioned and the organic layer was washed with saturated copper sulfate and water and then dried over MgSO₄ to give 5.9 g of the crude methyl esters. A solution of 5.72 g (21.0 mmol) of this mixture in 45 mL of anhydrous THF was treated with 400 mg (10.5 mmol) of lithium aluminum hydride. The reaction mixture was stirred at 25°C for 0.5 h and was then quenched by the slow addition of a saturated solution of Rochelle's salt. The mixture was diluted with ether, the layers were partitioned and the aqueous layer was washed twice with ethyl acetate. The combined organic extracts were dried over MgSO₄ and concentrated to give 4.9 g of the diastereomeric alcohols. Flash chromatography (elution with 1:5 ethyl acetate-hexane) gave 650 mg of (39), 1.10 g of (40) and 2.40 g of a mixture of the two. Data for (39): ¹H NMR (300 MHz, CDCl₃) δ 3.99-3.92 (m), 3.46 (d), 1.72-1.58 (m), 1.57-1.36 (m), 0.86 (s), 0.08 (s). Data for (40): ¹H NMR (300 MHz, CDCl₃)δ 3.47 (dddd), 3.38 (d), 1.86-1.67 (m), 1.47-1.16 (m), 1.05-0.77 (m), 0.72 (s), 0.02 (s).

### (E)-Ethyl 3-[trans-(4-tert-Butyldimethylsilyloxycyclohexyl)]-2-methylprop-2-enoate (41)

To a -78°C solution of oxalyl chloride (785 µL, 9.0 mmol) in 10 mL of methylene chloride was added dimethylsulfoxide (1.3 mL, 18.0 mmol). The resulting solution was stirred for 5 min and then 1.1 g (4.5 mmol) of the alcohol (40) was added in 10 mL of methylene chloride. The reaction mixture was stirred at -78°C for 45 min at which time 3.8 mL (27.0 mmol) of triethylamine was added and the solution was allowed to warm to ambient temperature. The reaction was quenched with 1.0 N HCl and the aqueous layer was extracted with three portions of methylene chloride. The combined organic extracts were dried over MgSO₄ and evaporated to dryness to give 1.0 g of the intermediate aldehyde. A solution of this aldehyde (450 mg, 1.86 mmol) was treated directly with 710 mg (1.95 mmol) of (carbethoxyethylidene)triphenylphosphorane in 5.0 mL of methylene chloride. The resulting reaction mixture was stirred at ambient temperature overnight and was then poured into water. The layers were partitioned and the aqueous layer was extracted twice with methylene chloride. The combined organic layers were dried over MgSO₄ and concentrated to yield the enoate (41) containing a minor amount of the Z isomer. ¹H NMR consistent with structure.

### (E)-3-[trans-(4-tert-Butyldimethylsilyloxycyclo-hexyl)]-2-methylprop-2-en-1-ol (42)

To a solution of 860 mg (2.6 mmol) of enoate (41) in 5.0 mL of anhydrous tetrahydrofuran at 25°C was added 50 mg (1.3 mmol) of lithium aluminum hydride and the resulting mixture was allowed to stir for 30 min. The reaction was quenched by the slow addition of saturated Rochelle's salt and diluted with ethyl acetate. The layers were separated and the aqueous layer was extracted with two portions of ethyl acetate. The combined organic extracts were washed with water and brine and then dried over MgSO₄. Evaporation and flash chromatography (elution with 15% ethyl acetate in hexane) gave 370 mg of the allylic alcohol (42). ¹H NMR consistent with structure.

### (E)-3-[trans-(4-tert-Butyldimethylsilyloxycyclo-hexyl)]-2-methylprop-2-en-1-0l (43)

To a -78°C solution of oxalyl chloride (105 µL, 1.2 mmol) in 1.0 mL of methylene chloride was added dimethylsulfoxide (170 µL, 2.4 mmol). The resulting solution was stirred for 5 min and then 170 mg (0.6 mmol) of the alcohol (42) was added in 1.0 mL of methylene chloride. The reaction mixture was stirred at -78°C for 45 min at which time 500 µL (3.6 mmol) of triethylamine was added and the solution was allowed to warm to ambient temperature. The reaction was quenched with 1.0 N HCl and the aqueous layer was extracted with three portions of methylene chloride. The combined organic extracts were dried over MgSO₄ and evaporated to dryness to give the crude aldehyde (43) which was used directly in the next reaction. ¹H NMR consistent with structure.

### (R and S)-(E)-1-[trans-(4-tert-Butyldimethylsilyloxycyclohexyl)]-2-methyl-6-phenyl-3-hex-1-en-3-ol (44)

The alcohol (44) was prepared from the crude aldehyde (43) and 1.5 mL (.075 mmol) of (27) in 2.0 mL of THF as described above for the synthesis of (28) in Example 1 to give 220 mg of the crude diastereomeric alcohol (44). Flash chromatography (elution with 20% ethyl acetate in hexane) afforded 146 mg of the alcohol (44) as an oil. ¹H NMR consistent with structure.

### (R and S)-(E)-1-[trans-(4-tert-Butyldimethylsilyloxycyclohexyl)]-2-methyl-6-phenyl-3-hex-1-enyl (S)-N-(3,4,5-trimethoxyphenylglyoxyl)pipecolate (45)

To a solution of 75.7 mg (0.22 mmol) of acid (34) in 2.5 mL of CH₂Cl₂ at room temperature was added 30 µL (0.34 mmol) of oxalyl chloride and three drops of DMF and the reaction mixture was allowed to stir at room temperature for 0.5 h and was then concentrated and suspended in 1.0 mL of benzene. To this suspension was added 43.4 mg (0.11 mmol) of alcohol (44) and 28.8 mg (0.22 mmol) of silver cyanide. The resulting mixture was heated at reflux overnight, cooled to room temperature and concentrated. Flash chromatography (elution with 4% acetone in hexane) gave 17.5 mg of the ester (45) as a mixture of diastereomers. ¹H NMR consistent with structure.

### (R and S)-(E)-1-[trans-(4-Hydroxycyclohexyl)]-2-methyl-6-phenyl-3-hex-1-enyl (S)-N-(3,4,5-trimethoxyphenylglyoxyl)pipecolate (8)

To a solution of 17.5 mg (0.02 mmol) of the ester (45) in 1.0 mL of CH₃CN at room temperature was added 10 drops of a 95:5 solution of CH₃CN:5% HF and the resulting mixture was stirred at room temperature for 0.5 h. The reaction mixture was neutralized with saturated K₂CO₃ and extracted into ether. The ether layers were washed with water, dried over MgSO₄ and concentrated to yield 7.2 mg of crude material. Flash chromatography (elution with 15% acetone in hexane) gave 4.9 mg of the diastereomeric alcohol (8) as a mixture of rotamers. ¹H NMR (500 MHz, CDCl₃)δ 7.38-7.02(m), 5.35-5.01(m), 4.62-4.53(m), 4.28(t), 3.95(s), 3.89(s), 3.87(s), 3.86(s), 3.85(s), 3.81(s), 3.55(m), 3.45(m), 3.20(m), 3.10-2.90(m), 2.60-2.45(m), 2.32(t), 2.10(t), 1.95(d), 1.85-1.40(m), 1.39-1.02(m).

### EXAMPLE 5

### Synthesis of (R and S)-5-(3-indolyl)-phenyl-2-pentyl (S)-N-(3,4,5-trimethoxyphenylglyoxyl)pipecolate (11)

### N-Methyl-N-Methoxy-4-(3-indolyl)butyramide (46)

To a slurry of 1.75 g (8.61 mmol) of 3-indolebutyric acid (Aldrich Chemical Co.) in acetonitrile at room temperature was added 7.0 mL (40.2 mmol) of N,N-diisopropylethylamine, 1.0 g (10.3 mmol) of N,N-dimethylhydroxylamine hydrochloride and 4.19 g (9.5 mmol) of benzotriazol-1-yloxy-tris(dimethylamino)phosphonium hexafluorophosphate (BOP reagent) and the resulting mixture was allowed to stir at room temperature overnight and was then concentrated to dryness. The residue was dissolved into ethyl acetate and washed with water, 0.5 N HCl, saturated NaHCO₃ and brine and then dried over MgSO₄ and concentrated. Flash chromatography (elution with a gradient of 2-10% ether in methylene chloride) provided 2.0 g of the amide (46). ¹H NMR consistent with structure.

### Benzyl-3-(3-indolyl)propyl ketone (47)

To a solution of 147 mg (0.60 mmol) of amide (46) in 4.0 mL of THF at -78°C was added 1.31 mL (1.31 mmol) of benzylmagnesium chloride (1.0 M in Et₂O) and the reaction mixture was allowed to warm to room temperature and stir for 3 h. The reaction was quenched with 5% KHSO₄ and extracted into ether. The combined ethereal layers were washed with brine and dried over MgSO₄. Flash chromatography (elution with 25% ether in hexane) gave 108 mg of the ketone (47). ¹H NMR consistent with structure.

### (R and S)-5-(3-indolyl)-1-phenyl-2-pentanol (48)

To a slurry of 105 mg (0.38 mmol) of ketone (47) in 3.0 mL of MeOH at 0°C was added 30 mg (0.79 mmol) of solid NaBH₄ and the resulting suspension was allowed to stir for 3 h. The reaction mixture was quenched with 5% KHSO₄ and extracted into ethyl acetate. The combined organic extracts were washed with brine and dried over MgSO₄. Flash chromatography (elution with 4% ether in methylene chloride) gave 81 mg of the alcohol (48) as a white solid. ¹H NMR consistent with structure.

### (S)-Boc-Pipecolyl-(R and S)-5-(3-indolyl)-1-phenyl-2-pentyl ester (49)

The ester (49) was prepared from 80 mg (0.29 mmol) of alcohol (48), 82 mg (0.36 mmol) of (S)-Boc-pipecolic acid, 66 mg (0.34 mmol) of EDC and a catalytic amount of 4-pyrrolidinopyridine in 2.0 mL of CH₂Cl₂ as described above for the synthesis of (29) in Example 1. Flash chromatography (elution with 4:10:26 ether:methylene chloride:hexane) gave 108 mg of the diastereomeric ester (49) as a white foam. ¹H NMR consistent with structure.

### (R and S)-5-(3-indolyl)-1-phenyl-2-pentyl (S)-pipecolate hydrochloride salt (50)

Anhydrous HCl was bubbled into a solution of 103 mg (0.21 mmol) of the ester (49) in 10 mL of EtOAc at -20°C for 10 min and then the reaction mixture was purged with N₂. Concentration gave 108 mg of the crude amine (50) as the hydrochloride salt. ¹H NMR consistent with structure.

### (R and S)-5-(3-indolyl)-1-phenyl-2-pentyl (S)-N-(3,4,5-trimethoxyphenylglyoxyl)pipecolate (11)

To a slurry of 108 mg of the crude amine hydrochloride (50) in CH₃CN at room temperature was added 91 µL (0.52 mmol) of N,N-diisopropylethylamine, 76 mg (0.31 mmol) of acid (31), and 111 mg (0.25 mmol) of the BOP reagent and the resulting mixture was stirred at room temperature for two days and was then concentrated to dryness. The residue was reconstituted into 75 mL of ethyl acetate and then sequentially washed with water, 5% KHSO₄, saturated NaHCO₃ and brine and then dried over MgSO₄ and concentrated. Flash chromatography (elution with 4% ether in methylene chloride) gave 56.7 mg of the diastereomeric amide (11) as a rotameric mixture. ¹H NMR (500 MHz, CDCl₃)δ 7.98 (d), 7.56 (t), 7.38-6.73 (m), 5.38-5.14 (m), 3.90 (m), 3.38 (brt), 3.10 (brt), 2.97-2.60 (m), 2.31 (d), 2.10 (d), 1.98-1.17 (m), 0.8 (m). R_{f} 0.51 (10% ether in methylene chloride).

### EXAMPLE 6

### Synthesis of (R and S)-2-Benzyl-4-phenyl-1-butyl (S)-N-(3,4,5-trimethoxyphenylglyoxyl)pipecolate (16)

### (R and S)-2-Benzyl-4-phenyl-1-butyric acid (51)

To a solution of 1.06 g (6.43 mmol) of 4-phenylbutyric acid in 20 mL of THF at 0°C was added 193 mg (6.43 mmol) of solid NaH (80% in mineral oil). After stirring at 0°C for 0.5 h, 3.2 mL (6.43 mmol) of lithium diisopropyl amide-THF complex (2.0 M) was added and the resulting red solution was stirred at 0°C for 45 min. To this mixture was added 765 µL (6.43 mmol) of benzylbromide and the solution was then allowed to stir overnight at room temperature. The reaction mixture was quenched by the slow addition of saturated NaHCO₃ and then washed with ether. The basic extracts were acidified with solid KHSO₄ and partitioned with ethyl acetate. The combined organic extracts were washed with brine, dried over MgSO₄ and concentrated to give 484 mg of the acid (51). ¹H NMR consistent with structure.

### (R and S)-2-Benzyl-4-phenyl-1-butanol (52)

To a solution of 469 mg (1.84 mmol) of acid (51) in 3.0 mL of THF at -78°C was added 2.03 mL (2.3 mmol) of lithium aluminum hydride (1.0 M in THF) and the resulting solution was allowed to warm to room temperature and stirred overnight. The reaction mixture was quenched by the slow addition of Rochelle's salt and partitioned with ether. The combined ether extracts were washed with water and brine and dried over MgSO₄ and concentrated. Flash chromatography (elution with 2% ether in methylene chloride) to afford 264 mg of the alcohol (52). ¹H NMR consistent with structure.

### (S)-Boc-Pipecolyl-(R and S)-2-Benzyl-4-phenyl-1-butyl ester (53)

The ester (53) was prepared from 264 mg (1.10 mmol) of alcohol (52), 302 mg (1.32 mmol) of (S)-Boc-pipecolic acid, 253 mg (1.32 mmol) of EDC and a catalytic amount of 4-pyrrolidinopyridine in 2.0 mL of CH₂Cl₂ as described above for the synthesis of (29) in Example 1. Flash chromatography (elution with 1:5:14 ether:methylene chloride:hexane) gave 375 mg of the diastereomeric ester (53). ¹H NMR consistent with structure.

### (R and S)-2-Benzyl-4-phenyl-1-butyl (S)-pipecolate hydrochloride salt (54)

Anhydrous HCl was bubbled into a solution of 375 mg (0.83 mmol) of the ester (53) in 10 mL of EtOAc at -20°C for 10 min and then the reaction mixture was purged with N₂. Concentration gave 352 mg of the crude amine (54) as the hydrochloride salt. ¹H NMR consistent with structure.

### (R and S)-2-Benzyl-4-phenyl-1-butyl (S)-N-(3,4,5-trimethoxyphenylglyoxyl)pipecolate (16)

To a slurry of 54 mg (0.14 mmol) of the crude amine hydrochloride (54) in 2.0 ml of CH₃CN at room temperature was added 60 µL (0.35 mmol) of N,N-diisopropylethylamine, 50 mg (0.21 mmol) of acid (31), and 73 mg (0.16 mmol) of the BOP reagent and the resulting mixture was stirred overnight at room temperature and was then concentrated to dryness. The residue was reconstituted into 75 mL of ethyl acetate and then sequentially washed with water, 5% KHSO₄, saturated NaHCO₃ and brine and then dried over MgSO₄ and concentrated. Flash chromatography (elution with 4% ether in methylene chloride) gave 52.7 mg of the diastereomeric amide (16) as a rotameric mixture. ¹H NMR (500 MHz, CDCl₃)δ 7.21-7.01 (m), 5.41 (brs), 4.21 (dd), 4.08 (dd), 4.12 (d), 3.88 (d), 3.95 (s), 3.91 (s), 3.49 (d), 3.39 (dt), 2.80-2.62 (m), 2.38 (brt), 2.09 (brs), 1.87-1.20 (m). R_{f} 0.9 (1:3:26 methanol:ether:methylene chloride).

### EXAMPLE 7

### Synthesis of (R and S)-1-Phenyl-7-(2-pyridyl)-4-heptyl (S)-N-(tert-butylglyoxyl)pipecolate (21)

### (E and Z)-3-(1,3-Dioxan-2-yl)-1-(2-pyridyl)-1-propene (55) and (56)

To a suspension of 4.6 g (10.2 mmol) of [2-(1,3-dioxan-2-yl)ethyl]triphenylphosphonium bromide (Aldrich Chemical Co.) in 50 mL of THF at 0°C was added 6.4 mL (10.2 mmol) of butyllithium (1.6 M in hexane) and the resulting red solution was allowed to stir at 0°C for 0.5 h. To this solution was added 880 µL (9.3 mmol) of 2-pyridinecarboxaldehyde (Aldrich Chemical Co.) and the reaction mixture was allowed to stir at room temperature for 1 h and was then poured into water and partitioned with ether. The combined ether extracts were dried over MgSO₄ and concentrated. Flash chromatography (elution with 3:1 hexane:ethyl acetate) gave 0.43 g of E-3-(1,3-dioxan-2-yl)-1-(2-pyridyl)-1-propene (55) and 1.12 g of Z-3-(1,3-dioxan-2-yl)-1-(2-pyridyl)-1-propene (56). ¹H NMR consistent with structures.

### 1-(1,3-Dioxan-2-yl)-3-(2-pyridyl)propane (57)

Through a solution of 800 mg (4.2 mmol) of olefin (56) and 100 mg of 10% palladium on carbon was bubbled in a steady stream of hydrogen gas for a period of 10 min. The reaction mixture was then filtered through celite and concentrated to give 805 mg of the acetal (57) as a colorless oil. ¹H NMR consistent with structure.

### 4-(2-Pyridyl)-butyraldehyde (58)

A solution of 420 mg (2.2 mmol) of acetal (57) in 4.0 mL of THF and 3.0 mL of 4N HCl was stirred at room temperature for 1.5 h and was then neutralized by the slow addition of solid NaHCO₃. The reaction mixture was extracted with ethyl acetate, dried over MgSO₄ and concentrated to yield 288 mg of the aldehyde (58). ¹H NMR consistent with structure.

### (R and S)-1-Phenyl-7-(2-pyridyl)-4-heptanol (59)

The alcohol (59) was prepared from 288 mg (1.93 mmol) of aldehyde (58) and 2.3 mL (2.3 mmol) of (27) in 3.0 mL of THF as described above for the synthesis of (28) in Example 1 to give 520 mg of the crude alcohol (59). ¹H NMR consistent with structure.

### (S)-Boc-Pipecolyl-(R and S)-1-phenyl-7-(2-pyridyl)-4-heptyl ester (60)

The ester (60) was prepared from 520 mg (1.93 mmol) of alcohol (59), 442 mg (1.93 mmol) of (S)-Boc-pipecolic acid, 370 mg (1.93 mmol) of EDC and a catalytic amount of DMAP in 4.0 mL of CH₂Cl₂ and 4.0 mL of DMF as described above for the synthesis of (29) in Example 1. Flash chromatography (elution with 3:1 hexane:ethyl acetate) gave 740 mg of the diastereomeric ester (60) as an oil. ¹H NMR consistent with structure.

### (R and S)-1-Phenyl-7-(2-pyridyl)-4-heptyl (S)-pipecolate (61)

The amine (61) was synthesized by treating 740 mg (1.54 mmol) of the ester (60) with 2.0 mL of trifluoroacetic acid in 5.0 mL of CH₂Cl₂ as described above for the preparation of (30) in Example 1 giving 580 mg of the diastereomeric amine (61) as an oil. ¹H NMR consistent with structure.

### (R and S)-1-Phenyl-7-(2-pyridyl)-4-heptyl (S)-N-methyloxalylpipecolate (62)

To a solution of 48 mg (0.13 mmol) of the amine (61) in 1.0 mL of CH₂Cl₂ at 0°C was added 33 µL (90.19 mmol) of N,N-diisopropylethylamine and 14 µL (0.15 mmol) of methyloxalyl chloride and the resulting solution was warmed to room temperature and allowed to stir overnight. The reaction mixture was diluted with ethyl acetate, washed with saturated NH₄Cl and brine, dried over MgSO₄ and then concentrated. Flash chromatography (elution with 25-30% ethyl acetate in hexane) gave 49 mg of the diastereomeric amide (62) as a mixture of rotamers. ¹H NMR consistent with structure.

### (R and S)-1-Phenyl-7-(2-pyridyl)-4-heptyl (S)-N-(tert-butylglyoxyl)-pipecolate (21)

To a solution of the amide (62) in 1.2 mL of THF at -78°C was added tert-butyl lithium dropwise until TLC showed the consumption of the starting material. The reaction mixture was quenched with saturated NH₄Cl and partitioned with ethyl acetate. The combined organic extracts were washed with brine, dried over MgSO₄ and concentrated. Flash chromatography (elution with 30% ethyl acetate in hexane) gave the diasteromeric amide (21) as a mixture of rotamers. ¹H NMR (500 MHz, CDCl₃)δ 8.50 (t), 7.57 (t), 7.20-7.05 (m), 5.23 (d), 5.18 (d), 4.56 (d), 4.44 (br d), 4.13 (d), 3.69 (br d), 3.37-3.28 (m), 3.13-3.00 (m), 2.85-2.70 (m), 2.65-2.54 (m), 2.38-2.15 (m), 1.82-1.65 (m), 1.56-1.44 (m), 1.55-1.30 (m), 1.27 (s), 1.21 (s).

### EXAMPLE 8

### Synthesis of (R and S)-1-Phenyl-7-(3-pyridyl)-4-heptyl (S)-N-(3,4,5-trimethoxyphenylglyoxyl)pipecolate N-oxide (22)

### (E and Z)-3-(1,3-Dioxan-2-yl)-1-(3-pyridyl)-propene (63)

To a suspension of 9.9 g (22.4 mmol) of [2-(1,3-dioxan-2-yl)ethyl]triphenylphosphoniumbromide (Aldrich Chemical Co.) in 50 mL of THF at 0°C was added 14.0 mL (22.4 mmol) of butyl lithium (1.6 M in hexane) and the resulting red solution was allowed to stir at 0°C for 0.5 h. To this solution was added 1.8 mL (18.7 mmol) of 3-pyridinecarboxaldehyde (Aldrich Chemical Co.) and the reaction mixture was allowed to stir at room temperature for 1.5 h and was then poured into water and partitioned with ether. The combined ether extracts were dried over MgSO₄ and concentrated. Flash chromatography (elution with 2:1 hexane:ethyl acetate) gave 3.3 g of the alkene (63) as a mixture of olefin isomers. ¹H NMR consistent with structure.

### 1-(1,3-Dioxan-2-yl)-3-(3-pyridyl)propane (64)

Through a solution of 3.2 g (16.7 mmol) of olefin (63) and 300 mg of 10% palladium on carbon was bubbled a steady stream of hydrogen gas for a period of 10 min. The reaction mixture was then filtered through celite and concentrated to give 2.8 g of the acetal (64) as a colorless oil. ¹H NMR consistent with structure.

### 4-(3-Pyridyl)-1-butyraldehyde (65)

A solution of 1.5 g (7.8 mmol) of acetal (64) in 10.0 mL of THF and 10.0 mL of 4N HCl was stirred overnight at room temperature and was then neutralized by the slow addition of solid NaHCO₃. The reaction mixture was extracted with ethyl acetate, dried over MgSO₄ and concentrated to yield 1.1 g of the aldehyde (65). ¹H NMR consistent with structure.

### (R and S)-1-Phenyl-7-(3-pyridyl)-4-heptanol (66)

The alcohol (66) was prepared from 1.1 g (7.4 mmol) of aldehyde (65) and 8.1 mL (8.1 mmol) of (27) in 30.0 mL of THF as described above for the synthesis of (28) in Example 1 to give 1.9 g of the crude alcohol (66). ¹H NMR consistent with structure.

### (S)-Boc-Pipecolyl-(R and S)-1-Phenyl-7-(3-pyridyl)-4-heptyl ester (67)

The ester (67) was prepared from 1.65 g (6.12 mmol) of alcohol (66), 1.54 g (6.73 mmol) of (S)-Boc-pipecolic acid, 1.29 g (6.73 mmol) of EDC and a catalytic amount of DMAP in 8.0 mL of CH₂Cl₂ and 8.0 mL of DMF as described above for the synthesis of (29) in Example 1. Flash chromatography (elution with 2:1 hexane:ethyl acetate) gave 1.42 g of the diastereomeric ester (67) as an oil. ¹H NMR consistent with structure.

### (R and S)-1-Phenyl-7-(3-pyridyl)-4-heptyl (S)-pipecolate (68)

The amine (68) was synthesized by treating 1.42 g (2.95 mmol) of the ester (67) with 2.0 mL of trifluoroacetic acid in 8.0 mL of CH₂Cl₂ as described above for the preparation of (30) in Example 1 giving 1.02 g of the diastereomeric amine (68) as an oil. ¹H NMR consistent with structure.

### (R and S)-1-Phenyl-7-(3-pyridyl)-4-heptyl (S)-N-(3,4,5-trimethoxyphenylglyoxyl)pipecolate (9)

The ester (9) was prepared from 995 mg (2.61 mmol) of the amine (68), 645 mg (2.87 mmol) of the acid (31) and 551 mg (2.87 mmol) of EDC in 6.0 mL of CH₂Cl₂ as described above in the synthesis of ester (3) in Example 1. Flash chromatography (elution with 3:1 acetone:hexane) gave 976 mg of the diasteromeric amide (9) as a mixture of rotamers. ¹H NMR consistent with structure.

### (R and S)-1-Phenyl-7-(3-pyridyl)-4-heptyl (S)-N-(3,4,5-trimethoxyphenylglyoxyl)pipecolate N-oxide (22)

To a solution of 15 mg (0.02 mmol) of the amide (9) in 2.0 mL of CH₂Cl₂ at room temperature was added 9.3 µL (0.03 mmol) of 55% 3-chloroperoxybenzoic acid and the resulting solution was allowed to stir overnight at room temperature. Flash chromatography (elution with 100% acetone) gave 12.6 mg of the N-oxide (22) as a mixture of rotamers. ¹H NMR (500 MHz, CDCl₃)δ 8.10(m), 7.46-7.02(m), 5.88(d), 5.80(d), 5.06-5.00(m), 4.95-4.89(m), 4.61(m), 4.31(dd), 3.87(s), 3.84(s), 3.83(s), 3.81(s), 3.78(s), 3.50(br d), 3.27(ddd), 3.12(ddd), 3.00(ddd), 2.67-2.49(m), 2.32(br d), 1.86-1.78(m), 1.55-1.50(m), 1.39-1.22(m).

### EXAMPLE 9

### Synthesis of (R and S)-1-Phenyl-7-purinyl-4-heptyl (S)-N-(3,4,5-trimethoxyphenylglyoxyl)pipecolate (25)

### 4-Chlorobutyraldehyde (69)

To a solution of 19.1 g (0.15 mmol) of 4-chloro-1-butanol (Aldrich Chemical Co.) in 50 mL of CH₂Cl₂ at 0°C was added 1.0 g of powedered 4A molecular sieves and 38.7 g (0.18 mmol) of pyridinium dichromate and the resulting suspension was stirred at 0°C for 45 min. The reaction mixture was diluted with ether, filtered through celite and concentrated. The residue was vacuum distilled (bp 45-55°C) to 5.0 g of the aldehyde (69) as an oil. ¹H NMR consistent with structure.

### (R and S)-1-Chloro-7-phenyl-4-heptanol (70)

The alcohol (70) was prepared from 182 mg (1.7 mmol) of aldehyde (69) and 1.9 mL (1.9 mmol) of (27) in 20.0 mL of THF as described above for the synthesis of (28) in Example 1 to give 128 mg of the crude alcohol (70). ¹H NMR consistent with structure.

### (S)-Boc-Pipecolyl-(R and S)-1-Chloro-7-phenyl-4-heptyl ester (71)

The ester (71) was prepared from 128 mg (0.56 mmol) of alcohol (70), 156 mg (0.68 mmol) of (S)-Boc-pipecolic acid, 130 mg (0.68 mmol) of EDC and a catalytic amount of 4-pyrrolidinopyridine in 2.0 mL of CH₂Cl₂ as described above for the synthesis of (29) in Example 1. Flash chromatography (elution with 1:5:14 ether:methylene chloride:hexane) gave 159 mg of the diastereomeric ester (71). ¹H NMR consistent with structure.

### (S)-Boc-Pipecolyl-(R and S)-1-Phenyl-7-purinyl-4-heptyl ester (72)

To a solution of 34 mg (0.28 mmol) of purine in 3.0 mL of DMF at room temperature was added 8.4 mg (0.28 mmol) of solid NaH (80% in mineral oil) and the resulting solution was allowed to stir at room temperature for 10 min. To this reaction mixture was added 62 mg (0.14 mmol) of the chloride (71) and 10 mg of NaI and this mixture was stirred overnight at room temperature and then concentrated to dryness. The residue was dissolved into ethyl acetate, washed sequentially with water, saturated NaHCO₃, and brine and then dried over MgSO₄ and concentrated. Flash chromatography (elution with 15% 5:10:85 NH₄OH:MeOH:CH₂Cl₂ in CH₂Cl₂) gave 56 mg of the substituted purine (72) as an oil. ¹H NMR consistent with structure.

### (R and S)-1-Phenyl-7-purinyl-4-heptyl (S)-pipecolate hydrochloride salt (73)

Anhydrous HCl was bubbled into a solution of 53.7 mg (0.10 mmol) of the ester (72) in 10 mL of EtOAc at -20°C for 10 min and then the reaction mixture was degassed with N₂. Concentration gave the crude amine (73) as the hydrochloride salt. ¹H NMR consistent with structure.

### (R and S)-1-Phenyl-7-purinyl-4-heptyl (S)-N-(3,4,5-trimethoxyphenylglyoxyl)pipecolate (25)

To a slurry of the crude amine hydrochloride (73) in CH₃CN at room temperature was added 45 µL (0.26 mmol) of N,N-diisopropylethylamine, 37 mg (0.15 mmol) of acid (31), and 54 mg (0.12 mmol) of the BOP reagent and the resulting mixture was stirred at room temperature for two days and then was concentrated to dryness. The residue was reconstituted into 75 mL of ethyl acetate and then sequentially washed with water, 5% KHSO₄, saturated NaHCO₃ and brine and then dried over MgSO₄ and concentrated. Flash chromatography (elution with 1:4:36 MeOH:Et₂O:CH₂Cl) gave 26.5 mg of the diastereomeric amide (25) as a rotameric mixture. ¹H NMR (500 MHz, CDCl₃)δ 9.11 (s), 8.95 (m), 8.09 (m), 7.36-7.05 (m), 5.31 (m), 4.28 (m), 3.90 (m), 3.46 (br t), 3.20 (m), 2.58 (m), 2.28 (br d), 2.17-1.18 (m). R_{f} 0.1 (30% ether in methylene chloride).

### DISCUSSION OF ASSAYS

### Cell Source and Culture

Fresh peripheral blood lymphocytes (PBLs) from LeukoPak cells or whole blood from random normal blood donors (tested HIV-negative and hepatitis negative) are isolated and separated by density centrifugation over Histopaque 1077 (Sigma Chemical Co., St. Louis, MO). The murine CTLL cytotoxic T cell line and the human Jurkat T cell line are from ATCC (CTLL-2 ATCC TIB214, JURKAT CLONE E6-1 ATCC TIBl52). The human allogeneic B cell lines used for activation of the fresh PBLs are EBV-transformed lymphocytes from normal healthy adult donors with two completely different HLA haplotypes. All cell lines were routinely tested for the presence of Mycoplasma contamination using the Gibco Mycotect test kit and are Mycoplasma-free. Culture medium consists of RPMI 1640 (Gibco, Grand Island, NY) containing penicillin (50 U/ml) and streptomycin (50 µg/ml), L-glutamine 2 mM, 2 mercaptoethanol (5 x 10⁻⁵), 10% heat-inactivated FCS and 10 mM HEPES.

### Compound Solutions and Titrations

All chemical stocks were dissolved in DMSO. Titrations of compounds were made into the medium the individual assay was carried out in, i.e., complete RPMI or HB 104 for final diluted concentrations, using multiple three-fold dilutions from 1 µM or 10 µM stock solutions.

### MTT Assay

The MTT assay is a colorimetric technique to determine the toxicity of the compounds on growing lymphoid and non-lymphoid cell lines based on reduction of the tetrazolium salt by intact mitochondria (Mossman, T., J. Immunol. Methods 65:55 (1983)). Cell viability in the presence or absence of different concentrations of test compounds in serum-free medium (HB 104, HANA Biologic, Inc.) was assessed using MTT (3-[4,5-dimethyl-thiazoyl-2-yl]2,5-diphenyl-tetrazolium bromide). At 4 h before the end of the 3-day toxicity assay culture period, 20 µl of MTT dye (5 mg/ml in pH 7.2 PBS) were added to each microtiter well. At the end of the incubation time, most of the culture media was carefully aspirated out of each well. Then 100 µl of acidified isopropyl alcohol (0.04 N HCl) was added to solubilize the dye and optical density is read at 570 nm minus OD at 630 nm (Molecular Devices Thermomax plate reader and Softmax software program, Menlo Park, CA). Results were compared with mean OD in controls (medium with no drugs) and doses causing 50% toxicity (TC₅₀) were calculated.

### Mitogenesis Assays ("PMA" and "OKT3")

The inhibitory effect of test compounds on the proliferation of human PBLs in response to mitogens (Waithe, W.K. and K. Hirschhorn, Handbook of Experimental Immunology, 3d Ed. Blackwell Scientific Publications, Oxford (1978); Mishell, B.B. and S.M. Shiigi, Selected Methods in Cellular Immunology W.H. Freeman and Co., San Francisco, CA (1980)) was assessed by stimulation of 5 x 10⁴ cells with OKT3 (10⁻⁴ dilution final) or PMA (10ng/ml) plus ionomycin (250 ng/ml) in the presence or absence of different concentrations of test compounds and control drugs (CsA, FK506, Pagamycin) in final volume of 200 µl per well in 96 well round bottomed plates. After 48 h incubation (37°C, 5% CO₂), cells were pulsed with 1 µCi of ³H-thymidine, harvested 24 h later with a Tom Tek cell harvester, and counted in LKB β-scintillation counter. Results (cpm) were compared with controls with medium alone, and concentrations causing 50% reduction in counts (IC₅₀) were calculated.

### MLR Bioassays ("LB" and "JVM")

Antigen activated proliferation of PBLs in a primary mixed lymphocyte reaction was assessed in the presence or absence of different concentrations of tested compounds and control drugs. 5 x 10⁴ fresh PBLs were stimulated with 5 x 10³ of Mitomycin C treated-allogeneic EBV-transformed β-lymphoblastoid cells, LB and JVM, in a final volume of 200 µl per well in 96-well round-bottomed plates (Mishell, B.B. and S.M. Shiigi, Selected Methods in Cellular Immunology W.H. Freeman and Co., San Francisco, CA (1980); Nelson, P.A. et al., Transplantation 50:286 (1990)). Cultures were pulsed on day 6, harvested 24 h later and counted as in previous section.

### IL-2 Microassay ("CTLL")

To determine if test compounds inhibit the later T cell activation process of cytokine utilization, the proliferative response of the IL-2 dependent CTLL-20 murine T cell line (ATCC) was assessed (Gillis, S. et al., J. Immunology 120:2027 (1978)). CsA and FK506 inhibit the production of IL-2 by activated T cells, whereas Rapamycin interferes with the utilization of IL-2. Rapamycin thus inhibits IL-2 dependent proliferation of the CTLLs, and CsA and FK506 do not (Dumont, F.J. et al., J. Immunology 144:251 (1990)). 3 x 10³ CTLLs were exposed to different concentrations of test compounds and control drugs in the presence of 1 U/ml of human recombinant IL-2 (Genzyme, rIL-2) for 24 h. Four h after adding drugs, cells were pulsed with 1 µCI of ³H-thymidine, incubated for an additional 20 h (37°C, 5% CO₂), and then harvested and counted as previously described.

### Bioavailability:

Bioavailability of compound (20) was determined in rats. A single dose of 50 mg/kg was administered by oral gavage or intraperitoneal (IP) injection in a vehicle consisting of olive oil-10% ethanol. Thereafter, animals were sacrificed at 0.5, 1, 2, 4, 8 and 12 hr, blood was collected into sodium heparin and immediately frozen. Whole blood was extracted with acetonitrile-methanol (90/10 vol:vol) and the concentration of compound per ml of blood was determined by HPLC. Data indicate that IP administration yielded blood levels of 0.7µM, 22µM, 225µM, 45µM and 1µM at 0.5h, 1h, 2h, 4h, and 8h, respectively. After oral administration, blood levels of 0.5µM, 1µM, 2µM, 12µM, and 3µM were measured at .05h, 1h, 2h, 4h, and 8h, respectively.

The blood levels attained after IP administration indicate adsorption from the peritoneal cavity into the circulation with maintenance of the bioactive structure. Blood levels achieved are sufficient for induction of immunosuppression.

Those skilled in the art will recognize, or be able to ascertain, using no more than routine experimentation, many equivalents to the specific embodiments of the invention described herein. Such equivalents are intended to be encompassed by the following claims:

## Claims

1. A compound having immunosuppressive activity, represented by the formula: 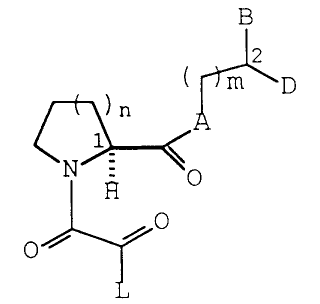 and pharmaceutically acceptable salts thereof, wherein A is O, NH, or N-(C1-C4 alkyl);
wherein B and D are independently Ar, (C5-C7)-cycloalkyl substituted (C1-C6)-straight or branched alkyl or (C2-C6)-straight or branched alkenyl, (C5-C7)-cycloalkenyl substituted (C1-C6)-straight or branched alkyl or (C2-C6)-straight or branched alkenyl, or Ar-substituted (C1-C6)-straight or branched alkyl, or Ar-substituted (C2-C6)-straight or branched alkenyl, wherein in each case, one or two carbon atoms of the straight or branched alkyl or alkenyl chains may be substituted with 1-2 heteroatoms selected from the group consisting of oxygen, sulfur, SO and SO₂, or 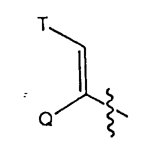
wherein Q is hydrogen, (C1-C6)-straight or branched alkyl or (C2-C6)-straight or branched alkenyl;
wherein T is Ar or substituted 5-7 membered cycloalkyl with substituents at positions 3 and 4 which are independently selected from the group consisting of hydrogen, oxo, hydroxyl, O-(C1-C4)-alkyl and O-(C2-C4)-alkenyl;
wherein Ar is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, monocyclic and bicyclic heterocyclic ring systems with individual ring sizes being 5 or 6 which may contain in either or both rings a total of 1-4 heteroatoms independently selected from oxygen, nitrogen and sulfur;
wherein Ar may contain one to three substituents which are independently selected from the group consisting of hydrogen, halogen, hydroxymethyl, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl and phenyl;
wherein L is U;
wherein U is O-(C1-C4)-straight or branched alkyl, O-(C2-C4)-straight or branched alkenyl, (C1-C6)-straight or branched alkyl, (C2-C6)-straight or branched alkenyl, (C5-C7)-cycloalkyl, (C5-C7)-cycloalkenyl substituted (C1-C4)-straight or branched alkyl, (C5-C7)-cycloalkenyl substituted (C2-C4)-straight or branched alkenyl, [(C1-C4)-alkyl or (C2-C4)-alkenyl]-Ar or Ar (Ar as described above);
wherein n is 1 or 2;
wherein m is 0 or 1; and
wherein the stereochemistry at carbon positions 1 and 2 are independently (R) or (S).

2. The immunosuppressant compound of Claim 1, wherein said compound has an affinity for FK-506 binding protein.

3. The immunosuppressant compound of Claim 2, wherein said compound is capable of inhibiting the prolyl peptidyl cis-trans isomerase activity of the FK-506 binding protein.

4. The immunosuppressant compound of any one of Claims 1 to 3, having a molecular weight of less than 750 amu.

5. The immunosuppressant compound of Claim 4, having a molecular weight of less than 500 amu.

6. The immunosuppressant compound of any one of Claims 1 to 5, wherein the stereochemistry at carbon position 1 is S.

7. The immunosuppressant compound of any one of Claims 1 to 6, wherein B is selected from the group consisting of 3-(2-pyridyl)propyl, 3-phenylpropyl, 2-phenoxyphenyl, phenyl, 2-(3-pyridyl)ethyl, E-3-[trans-(4-hydroxycyclohexyl)]-2-methyl-prop-2-enyl, 3-(3-pyridyl)propyl, benzyl, 2-phenylethyl, 2-(4-methoxyphenyl)ethyl, 3-(N-benzimidazoly)propyl, 3-(4-methoxyphenyl)propyl, 3-[N-(7-azaindolyl)propyl, 3-(N-purinyl)propyl, 3-(3-pyridyl)-N-oxide, 3-(4-hydroxymethylphenyl)propyl, 3-(2-thienyl)propyl, 3-(4-carboxyphenyl)propyl, 4-phenylbutyl, 2-hydroxymethylphenyl, 2-allyloxyphenyl, 3-(3-hydroxymethylphenyl)propyl, 3-(3-carboxyphenyl) propyl, 3-hydroxymethylphenyl, 2-hydroxyphenyl, 3-pyridyl and 5-phenylpentyl;
D is selected from the group consisting of 3-phenylpropyl, 2-phenoxyphenyl, 3-(3-indolyl)propyl, 2-phenylethyl, 4-phenylbutyl and 3-(4-methoxyphenyl)propyl; and
L is selected from the group consisting of 3,4,5-trimethoxyphenyl, phenyl, tert-butyl, 3-benzyloxyphenyl, 3-allyloxyphenyl and 3-isopropoxyphenyl.

8. A compound having immunosuppressive activity represented by any of the structures shown below and having an affinity for FK-506 binding protein:

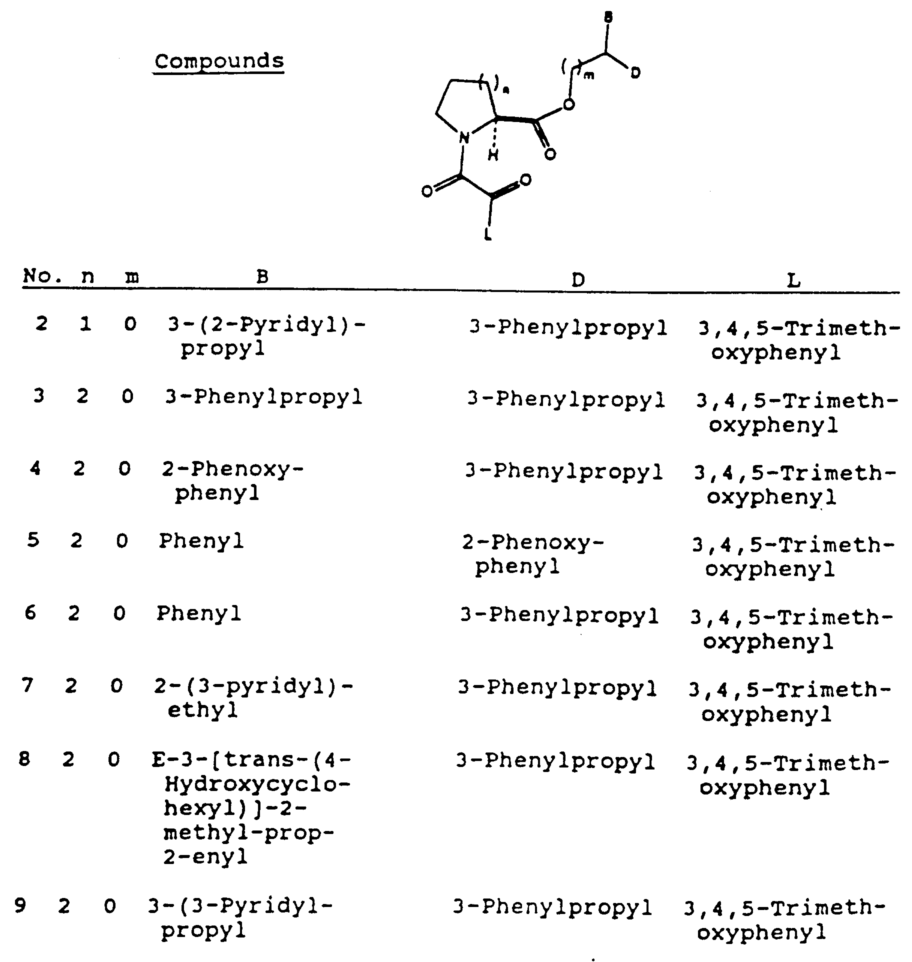

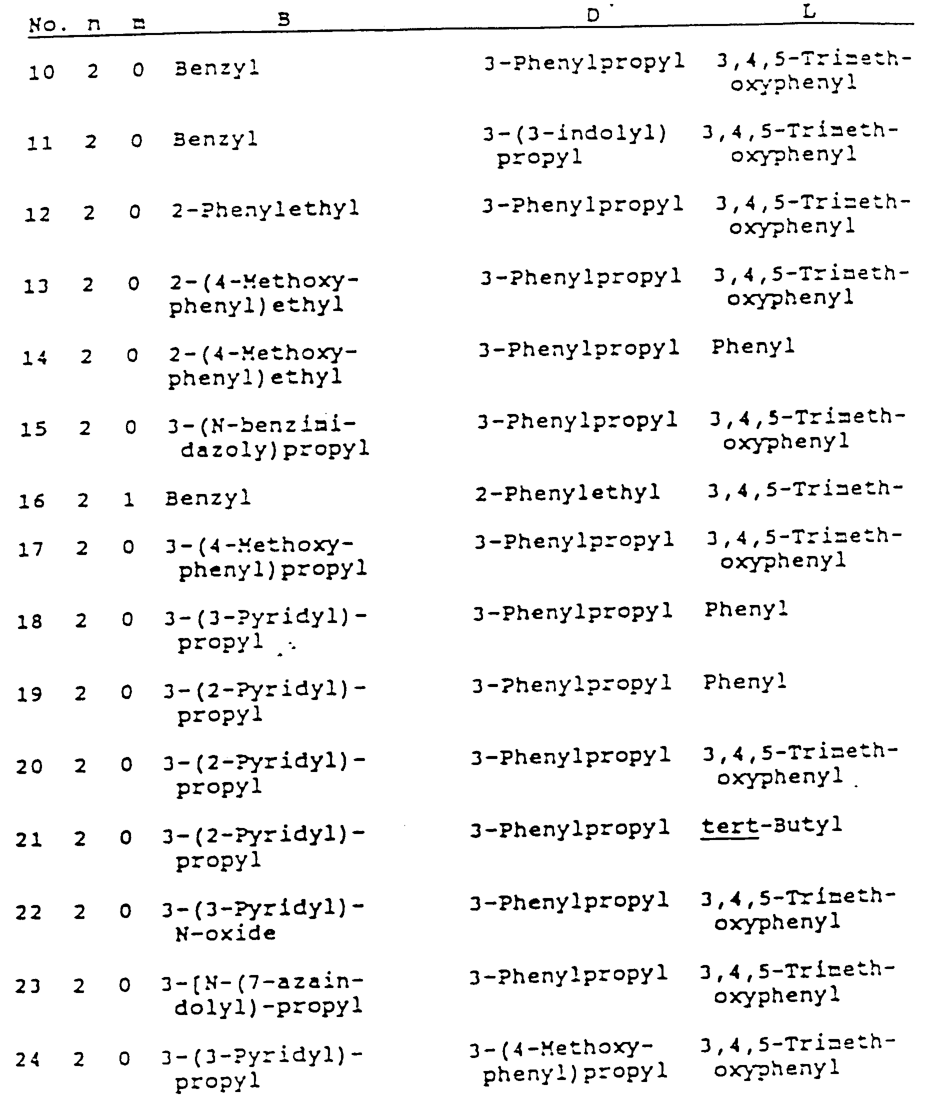

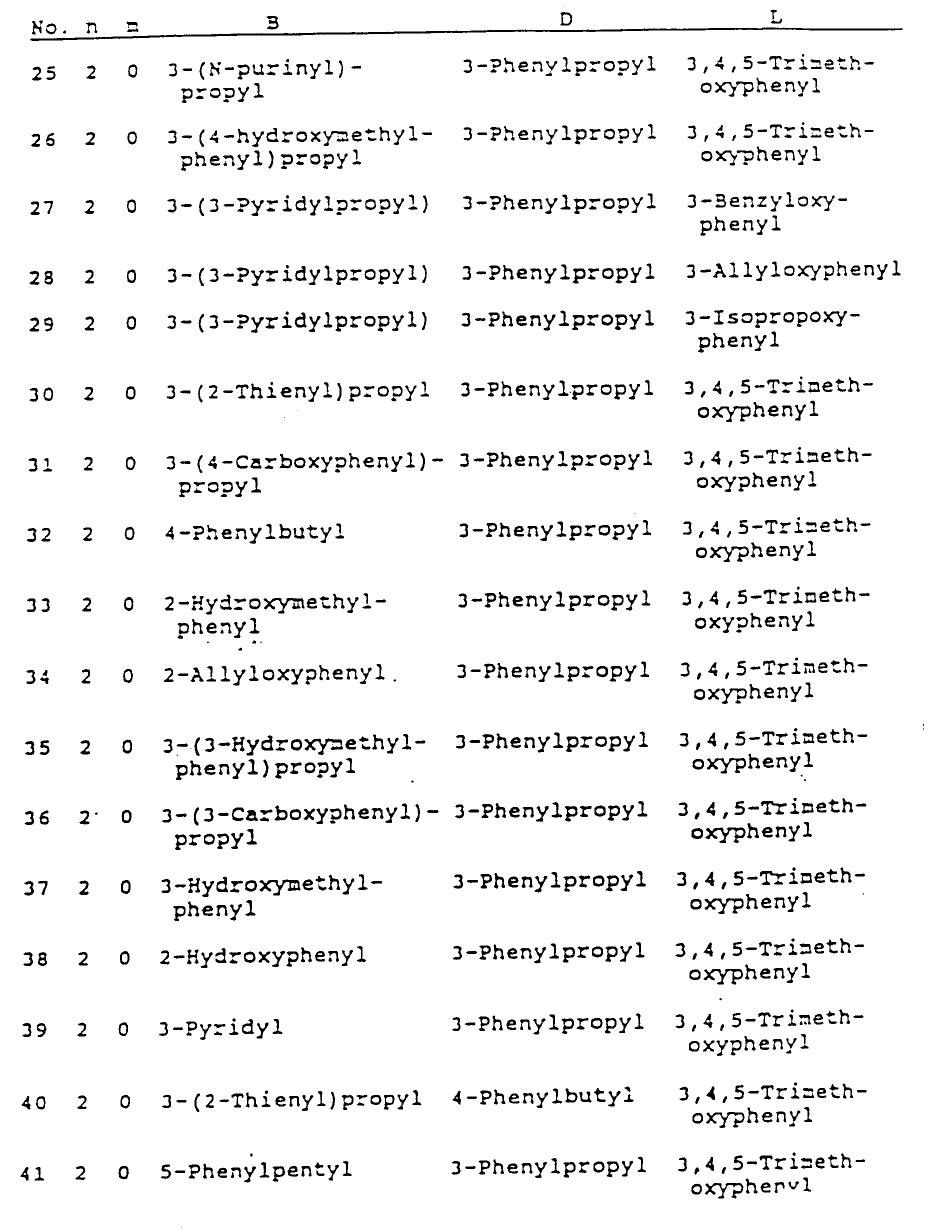

9. A composition for use in suppressing an immune response in a mammal, comprising the immunosuppressant compound of any one of Claims 1 to 8 having an affinity for FK-506 binding protein and having a molecular weight below 750 amu, in a physiologically acceptable vehicle.

10. Use of the immunosuppressant compound of any one of Claims 1 to 8 having an affinity for FK-506 binding protein and having a molecular weight below 750 amu in a physiologically acceptable vehicle for the manufacture of a medicament for use in suppressing an immune response in a mammal.

11. The composition or use of Claim 9 or 10, wherein the immune response to be suppressed is an autoimmune response or an immune response associated with graft rejection.

12. The composition or use of any one of Claims 9, 10, or 11, further comprising an immunosuppressant selected from the group consisting of cyclosporin, rapamycin, FK506, 15-deoxyspergualin, OKT3 and azathioprine.

13. The composition or use of any one of Claims 9, 10, 11, or 12, further comprising a steroid.

14. A process for preparing the compound having immunosuppressive activity of any one of claims 1 to 8;
said process comprising the step of reacting a compound of formula A: 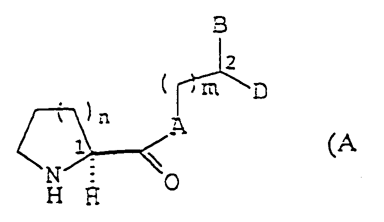
or a salt thereof with a compound of formula B: 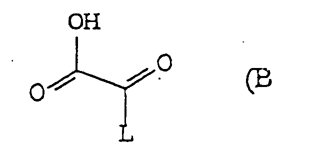
wherein A, B, D, L, m and n are as defined in claim 1.

15. A process for preparing the composition of claim 9 or 11 comprising the step of combining the compound having immunosuppressive activity of any one of claims 1 to 8 with a physiologically acceptable carrier, wherein said compound has an affinity for FK-506 binding protein and a molecular weight below 750 amu.

16. The process according to claim 15, wherein the immune response to be suppressed is an autoimmune response or an immune response associated with graft rejection.

17. The process according to claim 15 or 16 for preparing the composition of claim 12, further comprising the step of adding an immunosuppressant to said composition, wherein said immunosuppressant is selected from the group consisting of cyclosporin, rapamycin, 15-deoxyspergualin, FK506, OKT3 and azathioprine.

18. The process according to any one of claims 15 to 17 for preparing the composition of claim 13, further comprising the step of adding a steroid to said composition.

## Patentansprüche

1. Verbindung mit immunsuppressiver Wirkung, dargestellt durch die Formel 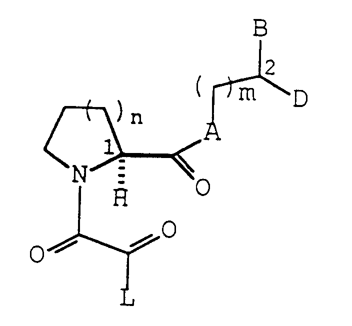 und pharmazeutisch verträgliche Salze davon,
wobei A ein Sauerstoffatom, eine NH-Gruppe oder ein N-(C₁-C₄-Alkyl)-Rest ist;
wobei B und D unabhängig voneinander einen Ar-, (C₅-C₇)-Cycloalkyl-substituierten (C₁-C₆)-geradkettigen oder verzweigten Alkylrest oder einen (C₂-C₆)-geradkettigen oder verzweigten Alkenylrest, einen (C₅-C₇)-Cycloalkenyl-substituierten (C₁-C₆)-geradkettigen oder verzweigten Alkylrest oder einen (C₂-C₆)-geradkettigen oder verzweigten Alkenylrest, oder einen Ar-substituierten (C₁-C₆)-geradkettigen oder verzweigten Alkylrest oder einen Ar-substituierten (C₂-C₆)-geradkettigen oder verzweigten Alkenylrest bedeuten, wobei in jedem Fall ein oder zwei Kohlenstoffatome der geradkettigen oder verzweigten Alkyl- oder Alkenylkette mit 1 bis 2 Heteroatomen substituiert sein können, ausgewählt aus Sauerstoff, Schwefel, SO- und SO₂-Gruppen, oder 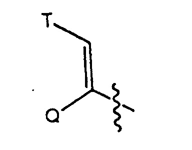 bedeutet, wobei Q ein Wasserstoffatom, ein (C₁-C₆)-geradkettiger oder verzweigter Alkylrest oder ein (C₂-C₆)-geradkettiger oder verzweigter Alkenylrest ist;
wobei T ein Ar-Rest oder ein substituierter 5- bis 7-gliedriger Cycloalkylrest mit Substituenten in den Stellungen 3 und 4 ist, die unabhängig voneinander ausgewählt sind aus Wasserstoffatomen, Oxo-, Hydroxy-, O-(C₁-C₄)-Alkyl- und O-(C₁-C₄)-Alkenylresten;
wobei Ar ausgewählt ist aus einer Phenyl-, 1-Naphthyl-, 2-Naphthyl-, 2-Furyl-, 3-Furyl-, 2-Thienyl-, 3-Thienyl-, 2-Pyridyl-, 3-Pyridyl-, 4-Pyridylgruppe, monocyclischen und bicyclischen heterocyclischen Ringsystemen mit individuellen Ringgrößen von 5 oder 6, die in einem oder beiden Ringen insgesamt 1 bis 4 Heteroatome enthalten können, unabhängig voneinander ausgewählt aus Sauerstoff, Stickstoff und Schwefel;
wobei Ar 1 bis 3 Substituenten enthalten kann, die unabhängig voneinander ausgewählt sind aus Wasserstoffatomen, Halogenatomen, Hydroxymethyl-, Hydroxyl-, Nitro-, Trifluormethyl-, Trifluormethoxy-, (C₁-C₆)-geradkettigen oder verzweigten Alkyl-, (C₂-₆)-geradkettigen oder verzweigten Alkenyl-, O-(C₁-C₄)-geradkettigen oder verzweigten Alkyl-, O-(C₂-C₄)-geradkettigen oder verzweigten Alkenyl-, O-Benzyl-, O-Phenyl-, 1,2-Methylendioxy-, Amino-, Carboxy- und Phenylresten;
wobei L den Rest U bedeutet;
wobei U einen O-(C₁-C₄)-geradkettigen oder verzweigten Alkyl-, O-(C₂-C₄)-geradkettigen oder verzweigten Alkenyl-, (C₁-C₆)-geradkettigen oder verzweigten Alkyl-, (C₂-C₆)-geradkettigen oder verzweigten Alkenyl-, (C₅-C₇)-Cycloalkyl-, (C₅-C₇)-Cycloalkenyl-substituierten (C₁-C₅)-geradkettigen oder verzweigten Alkyl-, (C₅-C₇)-Cycloalkenyl-substituierten (C₂-C₄)-geradkettigen oder verzweigten Alkenyl-, [(C₁-C₄)-Alkyl- oder (C₂-C₄)-Alkenyl]-Ar- oder Ar-Rest (wobei Ar wie vorstehend definiert ist) bedeutet;
wobei n den Wert 1 oder 2 hat,
wobei m den Wert 0 oder 1 hat, und
wobei die Stereochemie an den Kohlenstoffatomen 1 und 2 unabhängig voneinander (R) oder (S) ist.

2. Immunsuppressive Verbindung nach Anspruch 1, wobei die Verbindung eine Affinität für das FK-506-Bindungsprotein hat.

3. Immunsuppressive Verbindung nach Anspruch 2, wobei die Verbindung die Prolyl-peptidyl-cis-trans-Isomeraseaktivität des FK-506-Bindungsproteins hemmen kann.

4. Immunsuppressive Verbindung nach einem der Ansprüche 1 bis 3, mit einem Molekulargewicht von weniger als 750 Atommasseneinheiten (amu).

5. Immunsuppressive Verbindung nach Anspruch 4, mit einem Molekulargewicht von weniger als 500 amu.

6. Immunsuppressive Verbindung nach einem der Ansprüche 1 bis 5, wobei die Stereochemie an der Kohlenstoffposition 1 S ist.

7. Immunsuppressive Verbindung nach einem der Ansprüche 1 bis 6, wobei der Rest B ausgewählt ist aus 3-(2-Pyridyl)propyl, 3-Phenylpropyl, 2-Phenoxyphenyl, Phenyl, 2-(3-Pyridyl)ethyl, E-3-[trans-(4-Hydroxycyclohexyl)]-2-methyl-prop-2-enyl, 3-(3-Pyridyl)propyl, Benzyl, 2-Phenylethyl, 2-(4-Methoxyphenyl)ethyl, 3-(N-Benzimidazolyl)propyl, 3-(4-Methoxyphenyl)propyl, 3-[N-(7-azaindolyl)propyl, 3-(N-purinyl)propyl, 3-(3-Pyridyl)-N-oxid, 3-(4-Hydroxymethylphenyl)propyl, 3-(2-Thienyl)propyl, 3-(4-Carboxyphenyl)propyl, 4-Phenylbutyl, 2-Hydroxymethylphenyl, 2-Allyloxyphenyl, 3-(3-Hydroxymethylphenyl)propyl, 3-(3-Carboxyphenyl)propyl, 3-Hydroxymethylphenyl, 2-Hydroxyphenyl, 3-Pyridyl und 5-Phenylpentyl;
der Rest D ausgewählt ist aus 3-Phenylpropyl, 2-Phenoxyphenyl, 3-(3-Indolyl)-propyl, 2-Phenylethyl, 4-Phenylbutyl und 3-(4-Methoxyphenyl)propyl; und
der Rest L ausgewählt ist aus 3,4,5-Trimethoxyphenyl, Phenyl, tert.-Butyl, 3-Benzyloxyphenyl, 3-Allyloxyphenyl und 3-Isopropoxyphenyl.

8. Verbindung mit immunsuppressiver Wirkung dargestellt durch eine der Strukturen, die nachfolgend gezeigt sind, und mit einer Affinität für das FK-506-Bindungsprotein:

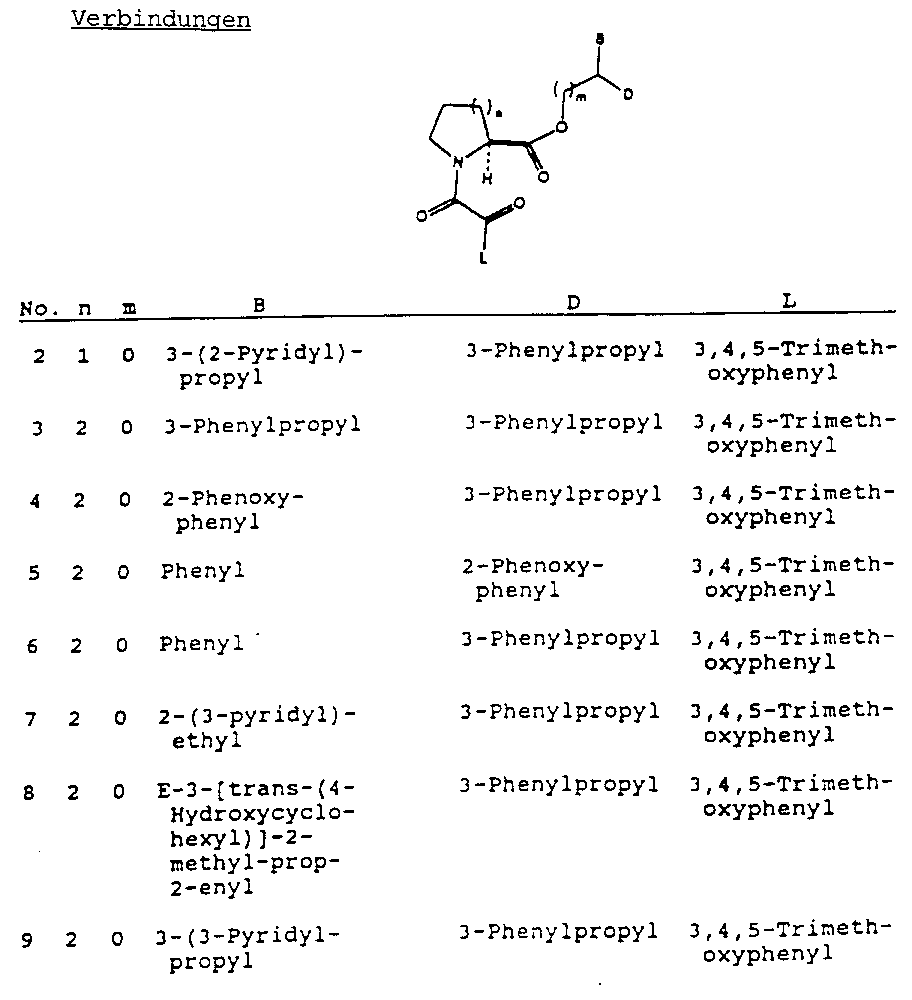

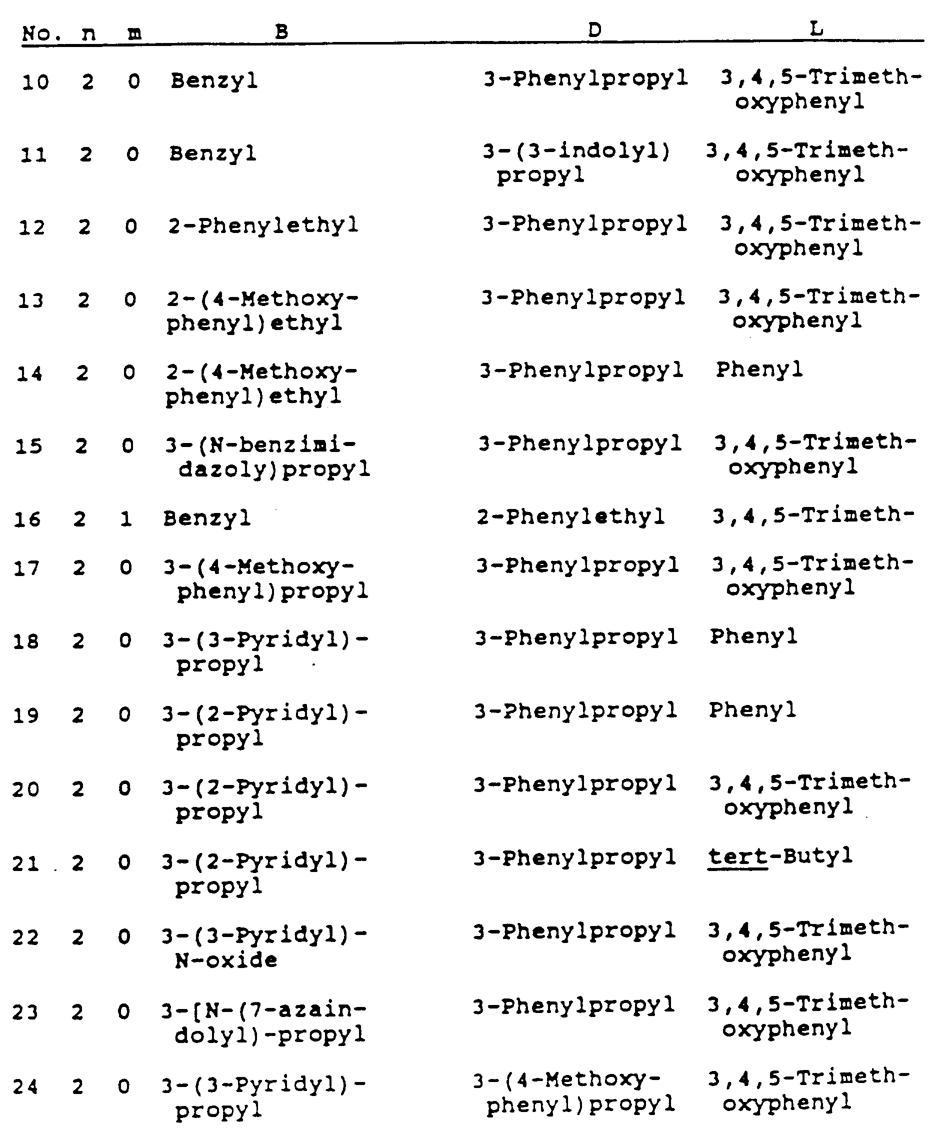

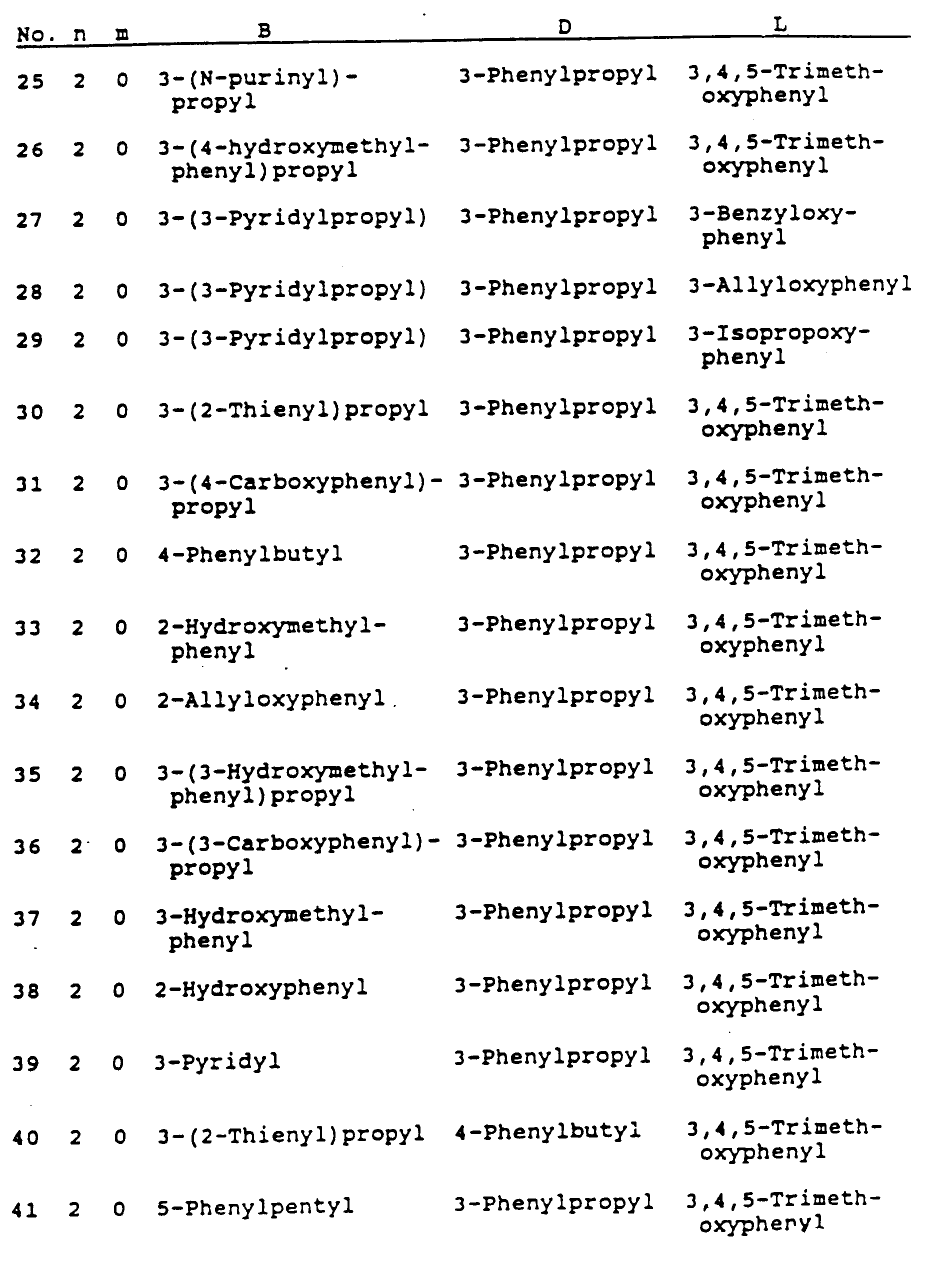

9. Zusammensetzung zur Verwendung in der Unterdrückung einer Immunantwort in einem Säuger, umfassend die immunsuppressive Verbindung nach einem der Ansprüche 1 bis 8 mit einer Affinität für das FK-506-Bindungsprotein und mit einem Molekulargewicht unter 750 amu, in einem physiologisch verträglichen Vehikel.

10. Verwendung der immunsuppressiven Verbindung nach einem der Ansprüche 1 bis 8 mit einer Affinität für das FK-506-Bindungsprotein und mit einem Molekulargewicht unter 750 amu in einem physiologisch verträglichen Vehikel für die Herstellung eines Medikaments zur Verwendung in der Unterdrückung einer Immunantwort in einem Säuger.

11. Zusammensetzung oder Verwendung nach Anspruch 9 oder 10, wobei die Immunantwort, die unterdrückt werden soll, eine Autoimmunantwort oder eine Immunantwort ist, die mit einer Transplantatabstoßung verbunden ist.

12. Zusammensetzung oder Verwendung nach einem der Ansprüche 9, 10 oder 11, zusätzlich ein Immunsupressivum umfassend, ausgewählt aus Cyclosporin, Rapamycin, FK506, 15-Desoxyspergualin, OKT3 und Azathioprin.

13. Zusammensetzung oder Verwendung nach einem der Ansprüche 9, 10, 11 oder 12, zusätzlich ein Steroid umfassend.

14. Verfahren zur Herstellung der Verbindung mit immunsuppressiver Wirkung nach einem der Ansprüche 1 bis 8,
wobei das Verfahren den Schritt der Umsetzung einer Verbindung der Formel A: 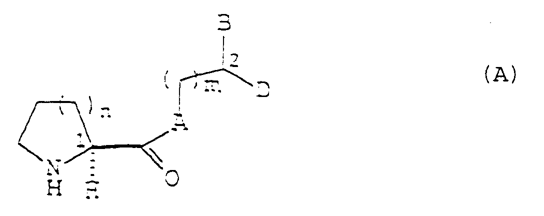
oder eines Salzes davon mit einer Verbindung der Formel B: 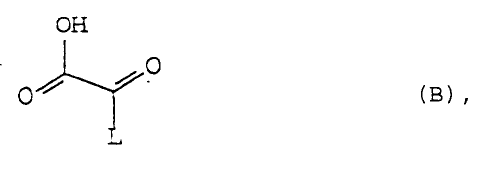
in der A, B, D, L, m und n wie in Anspruch 1 definiert sind, umfaßt.

15. Verfahren zur Herstellung der Zusammensetzung nach Anspruch 9 oder 11, umfassend den Schritt des Kombinierens der Verbindung mit immunsuppressiver Wirkung nach einem der Ansprüche 1 bis 8 mit einem physiologisch verträglichen Träger, wobei die Verbindung eine Affinität für das FK-506-Bindungsprotein und ein Molekulargewicht von weniger als 750 amu aufweist.

16. Verfahren nach Anspruch 15, wobei die zu unterdrückende Immunantwort eine Autoimmunantwort oder eine mit einer Transplantatabstoßung verbundene Immunantwort ist.

17. Verfahren nach Anspruch 15 oder 16 zur Herstellung der Zusammensetzung nach Anspruch 12, ferner umfassend den Schritt des Hinzufügens eines Immunsupressivums zu der Zusammensetzung, wobei das Immunsuppressivum Cyclosporin, Rapamycin, 15-Desoxyspergualin, FK506, OKT3 oder Azathioprin ist.

18. Verfahren nach einem der Ansprüche 15 bis 17 zur Herstellung der Zusammensetzung nach Anspruch 13, ferner umfassend den Schritt des Hinzufügens eines Steroids zu der Zusammensetzung.

## Revendications

1. Composé ayant une activité immunosuppressive, représenté par la formule : 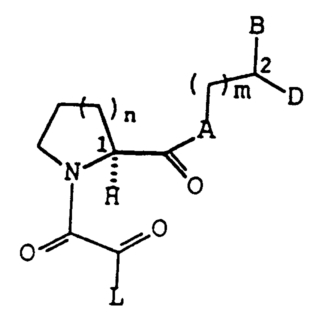 et ses sels pharmaceutiquement acceptables, dans laquelle A représente O, NH, ou N-alkyle en C1-C4 ;
dans laquelle B et D représentent indépendamment Ar, groupe alkyle en C1-C6 linéaire ou ramifié ou un groupe alcényle en C2-C6 linéaire ou ramifié substitué par un groupe cycloalkyle en C5-C7, un groupe alkyle en C1-C6 linéaire ou ramifié ou un groupe alcényle en C2-C6 linéaire ou ramifié substitué par un groupe cycloalcényle en C5-C7, ou un groupe alkyle en C1-C6 linéaire ou ramifié substitué par Ar, ou un groupe alcényle en C2-C6 linéaire ou ramifié substitué par Ar, où, dans chaque cas, un ou deux atomes de carbone des chaînes alkyle ou alcényle linéaires ou ramifiées peuvent être substitués par 1-2 hétéroatomes choisis dans l'ensemble constitue' d'atomes d'oxygène, de soufre, de SO et de SO₂, ou 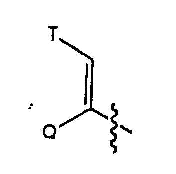
où Q représente un atome d'hydrogène, un groupe alkyle en C1-C6 linéaire ou ramifié ou un groupe alcényle en C2-C6 linéaire ou ramifié ;
où T représente Ar ou un groupe cycloalkyle à 5-7 membres substitué par des substituants en positions 3 et 4 qui sont indépendamment choisis dans l'ensemble constitué d'un atome d'hydrogène, d'un groupe oxo, hydroxyle, O-alkyle en C1-C4 et O-alcényle en C2-C4 ;
où Ar est choisi dans l'ensemble constitué des groupes phényle, 1-naphtyle, 2-naphtyle, 2-furyle, 3-furyle, 2-thiényle, 3-thiényle, 2-pyridyle, 3-pyridyle, 4-pyridyle, des systèmes de noyaux hétérocycliques monocycliques et bicycliques avec des tailles de noyaux individuels qui sont de 5 ou 6, qui peuvent contenir dans l'un ou les deux cycles un total de 1-4 hétéroatomes indépendamment choisis parmi les atomes d'oxygène, d'azote et de soufre ;
où Ar peut contenir un à trois substituants qui sont indépendamment choisis dans l'ensemble constitué d'atomes d'hydrogène, d'halogène, de groupes hydroxyméthyle, hydroxyle, nitro, trifluorométhyle, trifluorométhoxy, alkyle en C1-C6 linéaire ou ramifié, alcényle en C2-C6 linéaire ou ramifié, O-(alkyle en C1-C4 linéaire ou ramifié), O-(alcényle en C2-C4 linéaire ou ramifié), O-benzyle, O-phényle, 1,2-méthylènedioxy, amino, carboxyle et phényle ;
où L représente U ;
où U représente un groupe O-(alkyle en C1-C4 linéaire ou ramifié), O-(alcényle en C2-C4 linéaire ou ramifié), alkyle en C1-C6 linéaire ou ramifié, alcényle en C2-C6 linéaire ou ramifié, cycloalkyle en C5-C7, alkyle en C1-C4 linéaire ou ramifié substitué par un groupe cycloalcényle en C5-C7, alcényle en C2-C4 linéaire ou ramifié substitué par un groupe cycloalcényle en C5-C7, (alkyle en C1-C4 ou alcényle en C2-C4)-Ar ou Ar (Ar tel que décrit ci-dessus) ;
où n vaut 1 ou 2
où m vaut 0 ou 1 ; et
où la stéréochimie aux positions des atomes de carbone 1 et 2 est indépendamment (R) ou (S).

2. Composé immunosuppresseur de la revendication 1, ledit composé ayant une affinité pour la protéine de liaison FK-506.

3. Composé immunosuppresseur de la revendication 2, ledit composé étant capable d'inhiber l'activité de prolylpeptidyle cis-trans isomérase de la protéine de liaison FK-506.

4. Composé immunosuppresseur de l'une quelconque des revendications 1 à 3, ayant une masse moléculaire inférieure à 750 amu.

5. Composé immunosuppresseur de la revendication 4, ayant une masse moléculaire inférieure à 500 amu.

6. Composé immunosuppresseur de l'une quelconque des revendications 1 à 5, dans laquelle la stéréochimie à la position de l'atome carbone 1 est S.

7. Composé immunosuppresseur de l'une quelconque des revendications 1 à 6, dans lequel B est choisi dans l'ensemble constitué des groupes 3-(2-pyridyl)propyle, 3-phénylpropyle, 2-phénoxyphényle, phényle, 2-(3-pyridyl)éthyle, E-3-[trans-(4-hydroxycyclohexyl)]-2-méthyl-prop-2-ényle, 3-(3-pyridyl)propyle, benzyle, 2-phényléthyle, 2-(4-méthoxyphényl)éthyle, 3-(N-benzimidazoly)propyle, 3-(4-méthoxyphényl)propyle, 3-[N-(7-aza-indolyl)propyle, 3-(N-purinyl)propyle, 3-(3-pyridyl)-N-oxyde, 3-(4-hydroxyméthylphényl)propyle, 3-(2-thiényl)propyle, 3-(4-carboxyphényl)propyle, 4-phénylbutyle, 2-hydroxyméthylphényle, 2-allyloxyphényle, 3-(3-hydroxyméthylphényl)propyle, 3-(3-carboxyphényl)propyle, 3-hydroxyméthylphényle, 2-hydroxyphényle, 3-pyridyle et 5-phénylpentyle ;
D est choisi dans l'ensemble constitué des groupes 3-phénylpropyle, 2-phénoxyphényle, 3-(3-indolyl)-propyle, 2-phényléthyle, 4-phénylbutyle et 3-(4-méthoxyphényl)propyle ; et
L est choisi dans l'ensemble constitué des groupes 3,4,5-triméthoxyphényle, phényle, tert-butyle, 3-benzyloxyphényle, 3-allyloxyphényle et 3-isopropoxyphényle.

8. Composé ayant une activité immunosuppressive représentée par l'une quelconque des structures présentées ci-dessous et ayant une affinité pour la protéine de liaison FK-506.

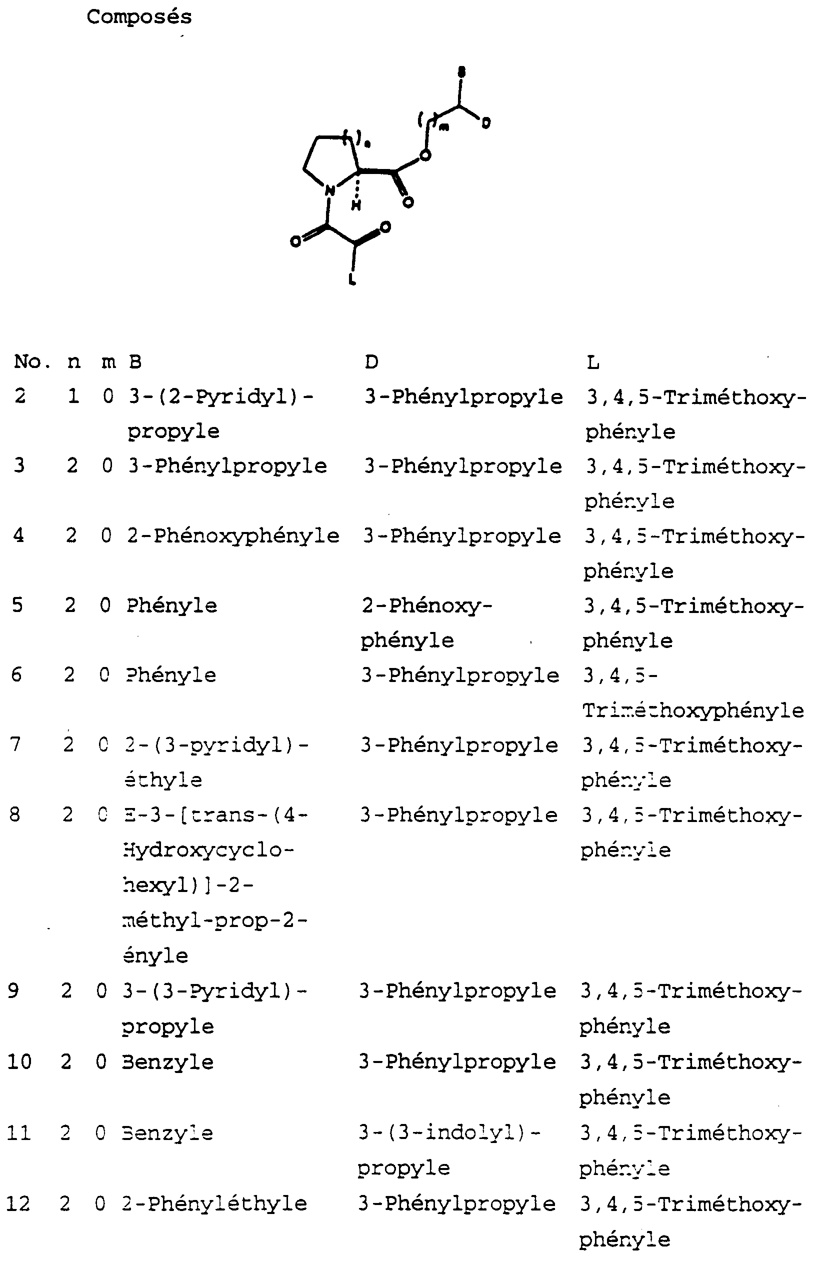

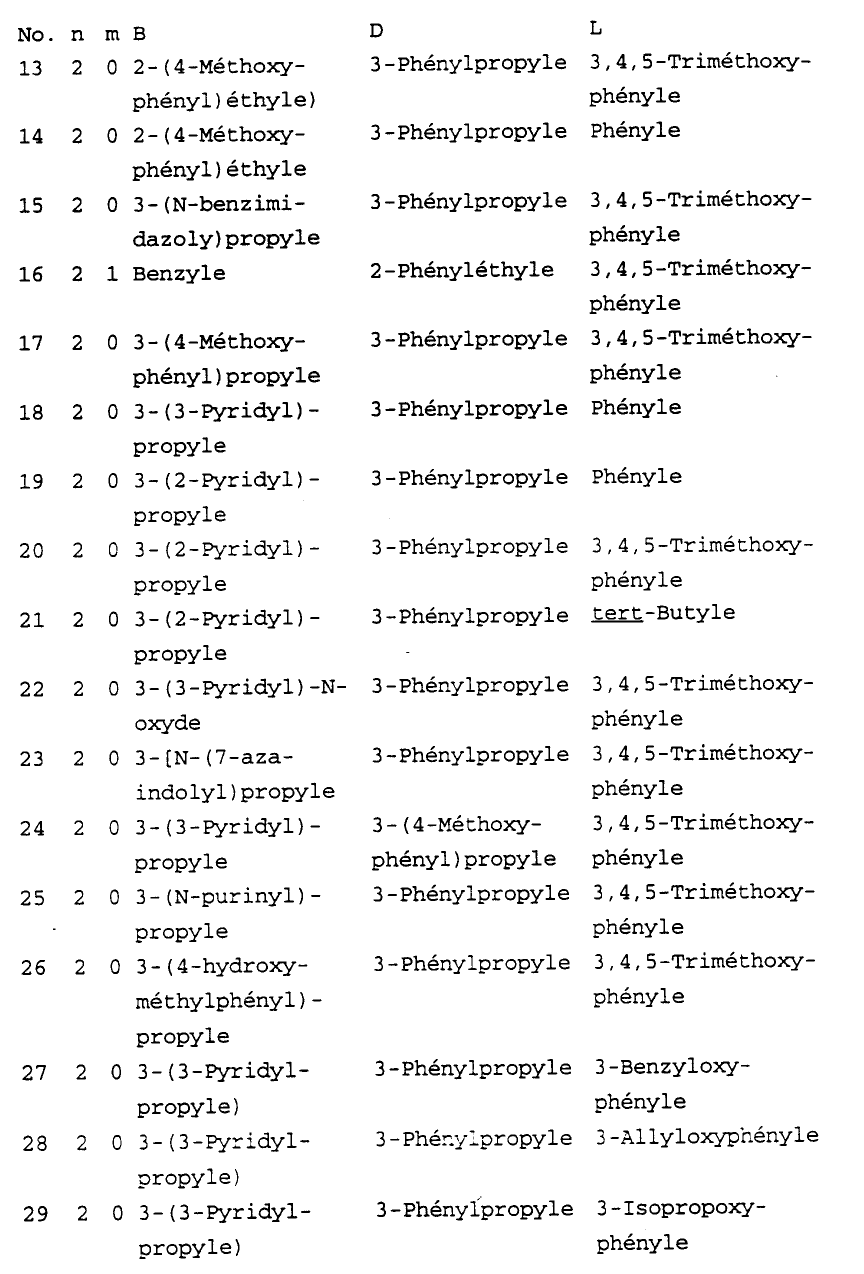

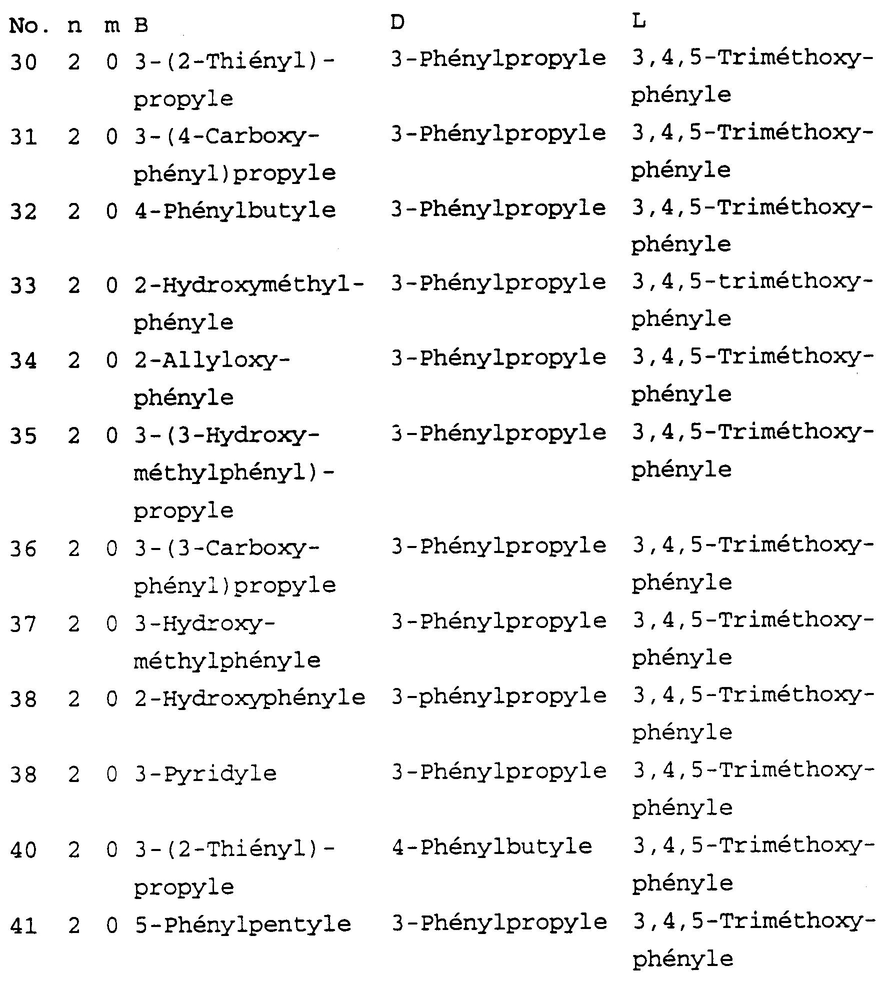

9. Composition destinée à être utilisée dans la suppression d'une réponse immunitaire chez un mammifère, comprenant le composé immunosuppresseur de l'une quelconque des revendications 1 à 8 ayant une affinité pour la protéine de liaison FK-506 et ayant une masse moléculaire inférieure à 750 amu, dans un véhicule physiologiquement acceptable.

10. Utilisation du composé immunosuppresseur de l'une quelconque des revendications 1 à 8, ayant une affinité pour la protéine de liaison FK-506 et ayant une masse moléculaire inférieure à 750 amu, dans un véhicule physiologiquement acceptable pour la fabrication d'un médicament destiné à être utilisé dans la suppression d'une réponse immunitaire chez un mammifère.

11. Composition ou utilisation de la revendication 9 ou 10, dans laquelle la réponse immunitaire à supprimer est une réponse auto-immune ou une réponse immunitaire associée avec un rejet de greffon.

12. Composition ou utilisation de l'une quelconque des revendications 9, 10 ou 11, comprenant en outre un immunosuppresseur choisi dans l'ensemble constitué de cyclosporine, rapamycine, FK506, 15-désoxyspergualine, OKT3 et azathioprine.

13. Composition ou utilisation de l'une quelconque des revendications 9, 10, 11 ou 12, comprenant en outre un stéroïde.

14. Procédé pour préparer le composé ayant l'activité immunosuppressive de l'une quelconque des revendications 1 à 8 ;
ledit procédé comprenant l'étape de réaction d'un composé de formule A : 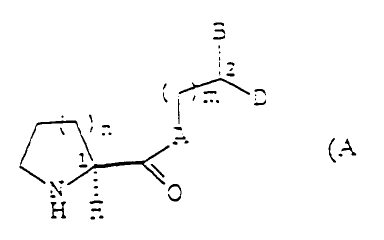
ou de l'un de ses sels avec un composé de formule B : 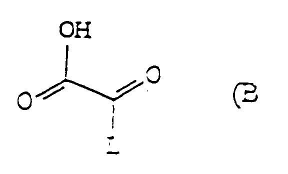
où A, B, D, L, m et n sont tels que définis dans la revendication 1.

15. Procédé de préparation de la composition de la revendication 9 ou 11, comprenant l'étape de combinaison du composé ayant une activité immunosuppressive de l'une quelconque des revendications 1 à 8 avec un véhicule physiologiquement acceptable, ledit composé ayant une affinité pour la protéine de liaison FK-506 et une masse moléculaire inférieure à 750 amu.

16. Procédé selon la revendication 15, dans lequel la réponse immunitaire à supprimer est une réponse auto-immune ou une réponse immunitaire associée avec un rejet de greffon.

17. Procédé selon la revendication 15 ou 16 pour la préparation de la composition de la revendication 12, comprenant en outre l'étape d'addition d'un immunosuppresseur à ladite composition, ledit immunosuppresseur étant choisi dans l'ensemble constitué de cyclosporine, rapamycine, 15-désoxyspergualine, FK506, OKT3 et azathioprine.

18. Procédé selon l'une quelconque des revendications 15 à 17 pour la préparation de la composition de la revendication 13, comprenant en outre l'étape d'addition d'un stéroïde à ladite composition.
